# EUROPEAN PATENT APPLICATION

(11) **EP 4 461 366 A1**
(43) Date of publication of application: **13.11.2024**
(21) Application number: 22918494.0
(22) Date of filing: 29.12.2022
(51) Int. Cl.: A61P 35/00, C12N 5/10, C07K 19/00, A61K 35/17, A61K 39/00

(54) **T CELL RECEPTOR, AND PREPARATION METHOD THEREFOR AND USE THEREOF**

(30) Priority: 05.01.2022 CN 202210005890
(71) Applicant: Suzhou Institute Of Systems Medicine, Suzhou, Jiangsu 215123 (CN)
(72) Inventor: LI, Guideng, Suzhou, Jiangsu 215123 (CN); CHENG, Hongcheng, Suzhou, Jiangsu 215123 (CN); QIU, Yajing, Suzhou, Jiangsu 215123 (CN); XU, Yue, Suzhou, Jiangsu 215123 (CN)
(74) Representative: ZBM Patents - Zea, Barlocci & Markvardsen
(86) International application number: PCT/CN2022/143567
(87) International publication number: WO 2023/131053

(57) **Abstract**

Construction of a T cell receptor and the use thereof are provided. Utilizing mutations of intracellular constant region sites of an α chain and an β chain of the T cell receptor to inhibit the degradation of the T cell receptor after TCR antigen signal activation, maintain the level of TCR on cell surface, and improve the efficacy of TCR-T cell therapy. This method can be applied to different TCR-T cell therapies.

## Description

### TECHNICAL FIELD

The present disclosure relates to the field of T cell receptors, and in particular, relates to the construction of a modified T cell receptor and the use thereof.

### BACKGROUND

The malignant tumor threatens human health and life and is one of the main causes of human death. The incidence rate thereof is increasing year by year. Traditional tumor therapy mainly relies on surgical treatment, supplemented by radiotherapy and chemotherapy. Compared with traditional therapy, tumor immunotherapy targets the immune system and stimulates systemic response to tumors. In addition, clinical data shows that tumor immunotherapy can significantly improve the prognosis of patients with specific types of tumors. The adoptive transfer of antigen-specific T cells is one of the main methods of tumor immunotherapy, which has been studied for the treatment of hematological and solid tumors. It is mainly based on the recognition of different tumor antigens by T cell receptors on the surface of T cells, thereby achieving the killing and clearance of tumor cells.

At present, the T cell adoptive therapy that has been extensively studied mainly includes CAR-T cell therapy and TCR-T therapy, wherein the CAR-T combines coupling antigen binding portions of antibodies that can recognize certain tumor antigens, and CD3-ζ chains to form chimeric proteins *in vitro,* then transfecting T cells of patients through gene transduction to express chimeric antigen receptors. When the T cells of patients are "re-encoded", many tumor-specific T cells are generated to achieve the effect of killing tumors. The TCR-T therapy is T cells recognizing the antigens presented by major histocompatibility complexes (MHC) on the surface of target cells through their surface TCR, thereby achieving direct attack and killing of target cells. It is a T cell therapy technology developed based on natural TCR or slight modifications to TCR. Due to its ability to recognize tumor epitopes presented by major histocompatibility complex molecules on the surface of tumor cells, it has a wider range of applicability.

The targetability of TCR-T cell therapy depends on the antigen-specific TCRs expressed on the surface of T cells. TCRs are receptors on the surface of T cells that bind to CD3 with non-covalent bonds and form TCR-CD3 complexes that activate T cells by recognizing and binding to antigens presented by MHC to promote the division and differentiation of T cells. Unlike CARs, most TCRs are heterodimers consisting of α chains and β chains, wherein α chains and β chains all contain antigen-binding regions, constant regions, and transmembrane domains. These α chains and β chains are covalently linked by disulfide bonds between conserved cysteine residues located within the fixed regions of each chain. Compared with CAR-T therapy, TCR-T can target most intracellular antigens, not limited to surface antigens, that is, TCR-T can target most tumor antigens, especially intracellular antigens that can recognize tumor cells. Therefore, the recognition range of T cell receptors for tumors is wider than that of antibody drugs, and CAR-T which rely on antibodies to recognize tumors. In addition, CAR-T has shortcomings in the treatment of solid tumors, and TCR-T technology is expected to solve this problem.

At present, a plurality of TCR-T optimization and modification methods are used to improve or enhance the effectiveness of TCR-T tumor treatment, which mainly enhances the recognition process of T cells towards tumor cells, enhances the affinity of T lymphocytes for tumor cells, and enables T cells that previously had no tumor recognition ability to effectively recognize and kill tumor cells, by modifying the "probe" - TCR of T cells binding to tumor antigens. Specifically include:
(1) Increase the expression of exogenous TCR to enhance the anti-tumor effect of TCR-T. a. Murinized TCR constant region: By utilizing genetic engineering techniques, the TCR constant region can be murinized to reduce or prevent a mismatch between endogenous and exogenous TCRs, promote preferential pairing of exogenous TCRs, increase the TCR expression level of T cell surface antigens, and thus enhance the anti-tumor effect of TCR-T. b. Cysteine insertion method of TCR constant region: Introducing cysteine into constant regions of TCR α chains and β chains to promote preferential pairing of exogenous TCRs, increase the expression level of exogenous TCRs, and reduce the mismatch with endogenous TCR chains. This method can improve the effectiveness and safety of tumor-reactive T cells, c. Inserted hydrophobic mutation in TCR transmembrane region: Increasing the hydrophobicity of TCR α chain transmembrane regions to improve the stability of TCRs, leading to enhanced expression of TCRs on the surface of T cells, thereby enhancing cell affinity and activity of anti-tumor TCRs.
(2) Increase the affinity between exogenous TCRs and tumor antigens. Synthetic T cell receptor and antigen receptor (STAR): Inserting the VH and VL of the antigen binding region of the antibody into the αβ constant region of TCR. This method not only has a high affinity like CAR but also has a high signal transduction ability like TCR complexes, thereby enhancing the antitumor effect of TCR-T.

Although modified TCR-T cell adoptive therapy has achieved good effects in the treatment of metastatic melanoma and other malignant tumors, there are still many limitations in the process of tumor treatment:
(1) T cell dysfunction. a. Continuous antigen stimulation within the tumor microenvironment leads to the depletion of CD8⁺ T cells, and the expression of inhibitory receptors such as PD-1, Lag-3, CD39, and TIM-3 is increased. b. The tumor microenvironment with low oxygen and low pH leads to progressive loss of T cell effector function, reduced secretion levels of proinflammatory cytokines such as IFN-γ and TNF-α, and decreased ability of T cell proliferation and self-renewal, and imbalanced metabolic activity.
(2) The expression level of TCR on the surface of T cells decreases. Antigen stimulation within the tumor microenvironment leads to decreased expression or accelerated degradation of exogenous TCR expression on the surface of CD8⁺ T cells, resulting in insufficient TCR-T cells attacking tumor cells.
(3) Therapeutic toxicity during clinical treatment. Currently, TCR-T-targeted antigens rarely have tumor tissue, therefore, tumor-targeted extra-tumoral toxicity may occur during the treatment process.

### SUMMARY

The problems to solve in the present disclosure

Considering some technical drawbacks of the TCR-T cell therapy described above, the purpose of the present disclosure is to provide a T cell receptor that can inhibit degradation and enhance the anti-tumor effect of TCR-T cells.

The solutions to solve the problems

On one aspect, the present disclosure provides a TCR α chain or fragment thereof isolated from a T cell receptor (TCR). The TCR α chain includes a TCR α chain constant region. The TCR α chain constant region includes a TCR α chain extracellular constant region, a TCR α chain transmembrane region, and a TCR α chain intracellular constant region that are connected in a sequential order. The TCR α chain intracellular constant region is a mutated TCR α chain intracellular constant region. The mutated TCR α chain intracellular constant region is formed by at least one serine mutated to alanine in a wild-type TCR α chain intracellular constant region.

On the other aspect, the present application provides a TCR β chain or fragment thereof isolated from a T cell receptor (TCR). The TCR β chain includes a TCR β chain constant region. The TCR β chain constant region includes a TCR β chain extracellular constant region, a TCR β chain transmembrane region, and a TCR β chain intracellular constant region that are connected in a sequential order. The TCR β chain intracellular constant region is a mutated TCR β chain intracellular constant region. The mutated TCR β chain intracellular constant region is formed by at least one lysine mutated to arginine or alanine in wild-type TCR β chain intracellular constant region.

On the other aspect, the present disclosure provides an isolated T cell receptor or fragment thereof. The T cell receptor includes any of the aforementioned TCR α chain and/or the aforementioned TCR β chain.

On the other aspect, the present disclosure provides one or more kinds of T cell receptors, such as F5-WT TCR, F5-SA TCR, F5-KR TCR, or F5-dMUT TCR, such as 1G4-WT TCR, 1G4-SA TCR, 1G4-KR TCR or 1G4-dMUT TCR.

On the other aspect, the present disclosure provides an isolated nucleic acid or fragment thereof, which encodes any of the aforementioned TCR α chain or fragment thereof isolated from the TCR, TCR β chain or fragment thereof isolated from the TCR, or T cell receptor or fragment thereof.

On the other aspect, the present disclosure provides a nucleic acid construct, which includes any of the aforementioned isolated nucleic acid or fragment thereof.

On the other aspect, the present disclosure provides a vector, which includes any of the aforementioned nucleic acid construct.

On the other aspect, the present disclosure provides an engineered cell, which includes any of the aforementioned nucleic acid construct or vector.

On the other aspect, the present disclosure provides a TCR complex, which is generated in T cells by any of the aforementioned TCR nucleic acid construct in the present disclosure.

On the other aspect, the TCR-T cells provided in the present disclosure undergo functional testing during *in vitro* culture.

On the other aspect, the present disclosure provides the application of any of the aforementioned TCR α chain isolated from the TCR or fragment thereof, TCR β chain or fragment thereof isolated from the TCR, T cell receptor or fragment thereof, nucleic acid or fragment thereof, nucleic acid construct, vector or engineered cell in any one or more of the following uses:
(1) Application of inhibiting T cell receptor degradation during antigen stimulation process;
(2) Providing the killing effect on tumors, or inhibiting tumor growth;
(3) Maintaining the proliferation ability of T cells;
(4) Preparation of anti-tumor immune drugs and cells.

On the other aspect, the mutated TCR-T cell therapy provided in the present disclosure can be used alone or in combination with other therapies, in combination with PD-1/PD-L1 antibody, or in combination with cytokine therapy, or combination with radiotherapy and chemotherapy.

On the other aspect, the present disclosure provides a modification method for the T cell receptor, comprising:
(1) Mutating at least one lysine in the wild-type TCR β chain intracellular constant region to arginine or alanine; and/or
(2) Mutating at least one serine in the wild-type TCR α chain intracellular constant region to or alanine.

The effect of the present disclosure

The present disclosure utilizes mutations in the ubiquitination modification sites of the α and β intracellular constant regions of the T cell receptor to inhibit the degradation of the T cell receptor after activation of TCR antigen signals, and maintain the level of TCR on the cell surface. This method may be applicable to different TCR-T cell therapies.

On one aspect, after antigen stimulation, compared to WT TCR cells (wild-type TCR cells), SA TCR, KR TCR, and dMUT TCR cells have more TCR expression on the cell surface. During the process of *in vitro* cultivation, SA TCR, KR TCR, and dMUT TCR cells exhibit phenotypes of differentiation towards central memory T cells. The *in vitro* exhaustion model shows that compared to WT TCR, KR TCR, and dMUT TCR cells have lower levels of exhaustion. The transformation of this phenotype is attributed to the healthier mitochondrial state within SA TCR, KR TCR, and dMUT TCR cells, which manifested as mutant TCR-T cells cultured *in vitro* exhibit lower mitochondrial membrane potential and ROS level. On the other aspect, the present disclosure finds that compared to WT TCR, KR TCR and dMUT TCR cells have stronger anti-tumor effects by Mouse transplanted tumor-T cell adoptive model. This present disclosure also finds that the dMUT TCR mutant group has more TCR-T cells present in the mouse spleen and blood, while in tumor tissues, the proportion of KR TCR and dMUT TCR cells significantly increased. From a cellular phenotype perspective, SA TCR, KR TCR, and dMUT TCR groups present in the mouse spleen accumulated more central memory T cells, while TCR-T cells with dMUT mutations in tumor tissue exhibited lower T cell exhaustion phenotype and proliferation ability. Therefore, in the process of adopting equal amounts of TCR-T cells for treatment, KR TCR and dMUT TCR cells exhibit better potential for inhibiting tumor growth.

It can be understood that the present disclosure not only inhibits TCR degradation but also improves the efficacy of TCR-T cell therapy by the mutation of amino acids in TCR intracellular constant regions, which can effectively inhibit tumor growth. The tumor is not limited to solid tumors and can be hematological tumors or lymphomas.

### BRIEF DESCRIPTIONS OF THE DRAWINGS

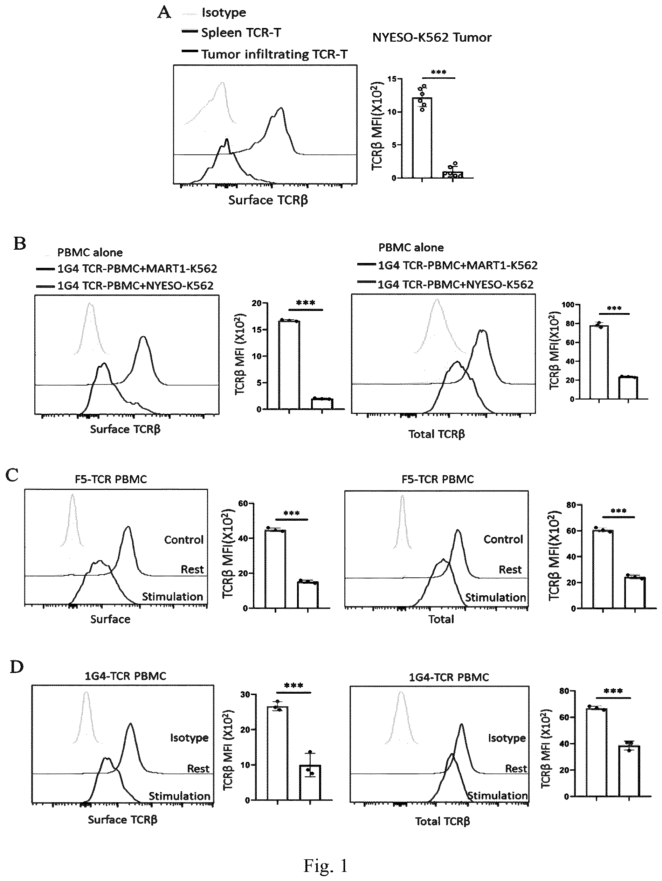

FIG.1 shows that antigen stimulation promotes the degradation of TCR on the cell surface. (A) On the twelfth day after 1G4-TCR-T cell adoption in tumor-bearing mice inoculated with K562-NYESO-1, detecting the expression of TCR on the surface of TCR-T cells in spleens and tumors. (B) *In vitro* functional experiments are conducted on human primary T cells. 1G4-TCR-T cells are cultured *in vitro* and co-cultured separately with K562-NYESO-1 and K562-MART-1 cells for 12 hours to detect the degradation of TCR. (C) Activated F5-TCR-T cells are cultured *in vitro.* Detecting the degradation of TCR after 12 hours of CD3 antibody stimulation. (D) Activated 1G4-TCR-T cells are cultured *in vitro.* Detecting the degradation of TCR after 12 hours of CD3 antibody stimulation.

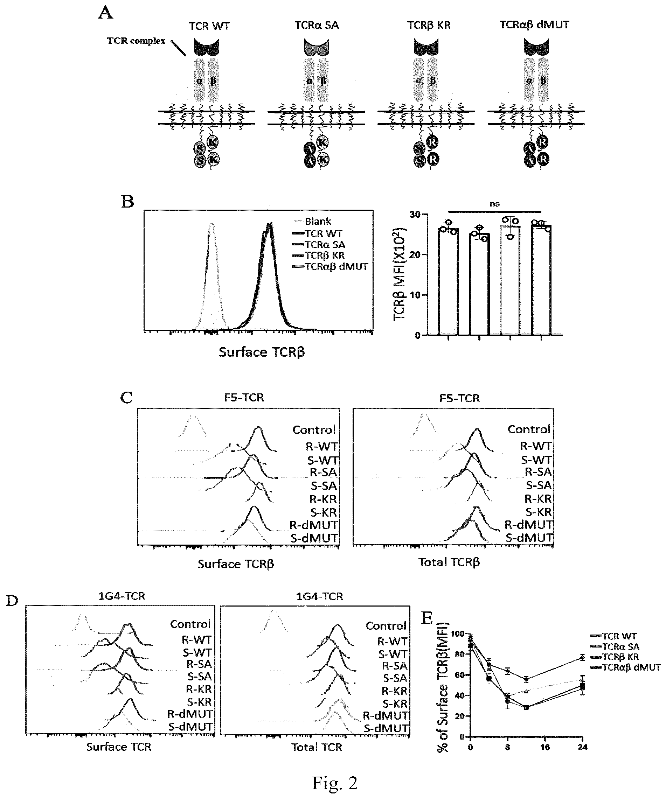

FIG. 2 is a resulting graph of TCR downregulation and degradation inhibited by mutations. *In vitro* functional experiments are conducted on human primary T cells. (A) Mutation pattern diagram of degradation inhibited TCR. (B) Expression of mutated 1G4-TCR on human primary T cells. (C) Activated F5-TCR-T cells with different mutations *in vitro.* Detecting the degradation of different TCRs after 12 hours of CD3 antibody stimulation ("R" represents no CD3 antibody stimulation, "S" represents CD3 antibody stimulation). (D) Activated 1G4-TCR-T cells with different mutations *in vitro.* Detecting the degradation of different TCRs after 12 hours of CD3 antibody stimulation ("R" represents no CD3 antibody stimulation, "S" represents CD3 antibody stimulation). (E) Activated 1G4-TCR-T cells with different mutations *in vitro.* Detecting the degradation of different TCRs after different times of CD3 antibody stimulation.

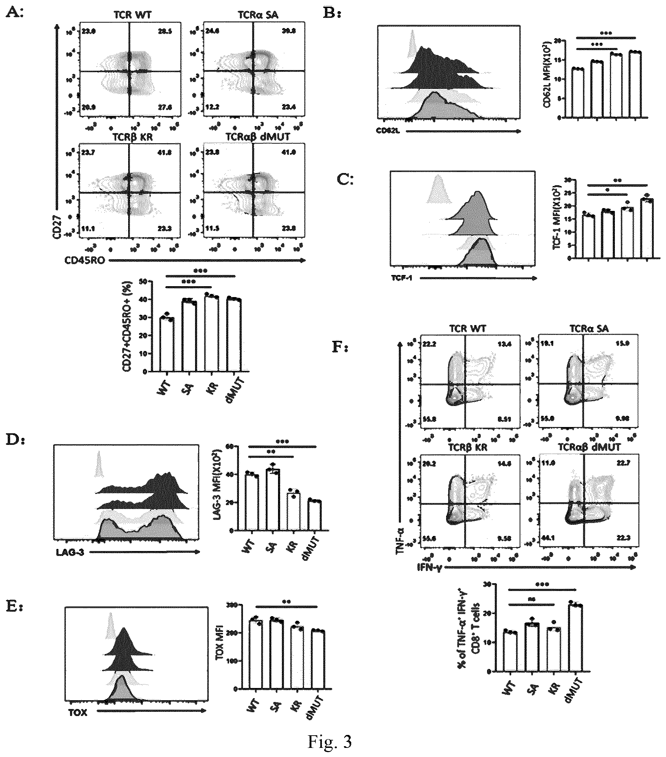

FIG. 3 shows effect of mutations on the *in vitro* function of 1G4-TCR-T cells. *In vitro* functional experiments are conducted on human primary T cells. (A) TCR-T cells activated *in vitro* are subjected to flow cytometry analysis using CD45RO and CD27 as differentiation indicators for memory T cells. (B) Activated 1G4-TCR-T cells. The indicator for detecting memory T cell differentiation is CD62L. (C) 1G4-TCR-T cells activated *in vitro.* The indicator for detecting memory T cell differentiation is TCF-1. (D) Using an *in vitro* exhaustion model, detect the expression of exhaustion molecule LAG-3 in different mutant TCR-T cells. (E) Using an *in vitro* exhaustion model, detect the expression of key transcription factor TOX in TCR-T cell exhaustion. (F) Using an *in vitro* exhaustion model, detect the production of cytokines in mutant TCR-T.

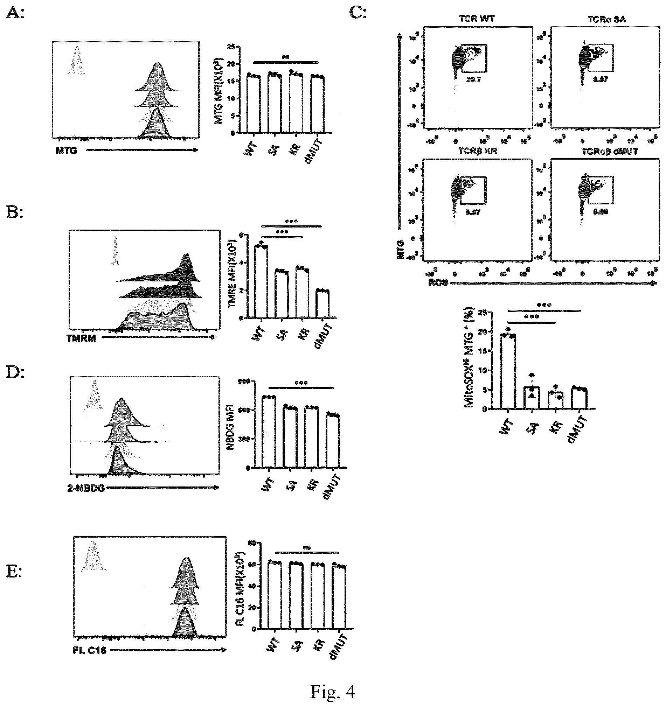

FIG. 4 is a comparison chart of mitochondrial functional status in 1G4-TCR-T cells with different mutations. *In vitro* experiments are conducted on human primary T cells. (A) Detecting the number of intracellular mitochondria in different TCR-T cells after 12 days of *in vitro* activation. (B) Detecting the changes in mitochondrial membrane potential in mutated 1G4-TCR-T cells. (C) 1G4-TCR-T cells activated *in vitro.* Detecting the production level of intracellular mitochondrial ROS. (D) After 12 days of *in vitro* activation of different TCR-T cells and treatment with 2-NBDG, FACS flow cytometry was used to detect glucose uptake by TCR-T cells. (E) Bodipy FL C16 uptake experiment was used to detect the utilization efficiency of extracellular fatty acids by TCR-T cells in different mutation groups.

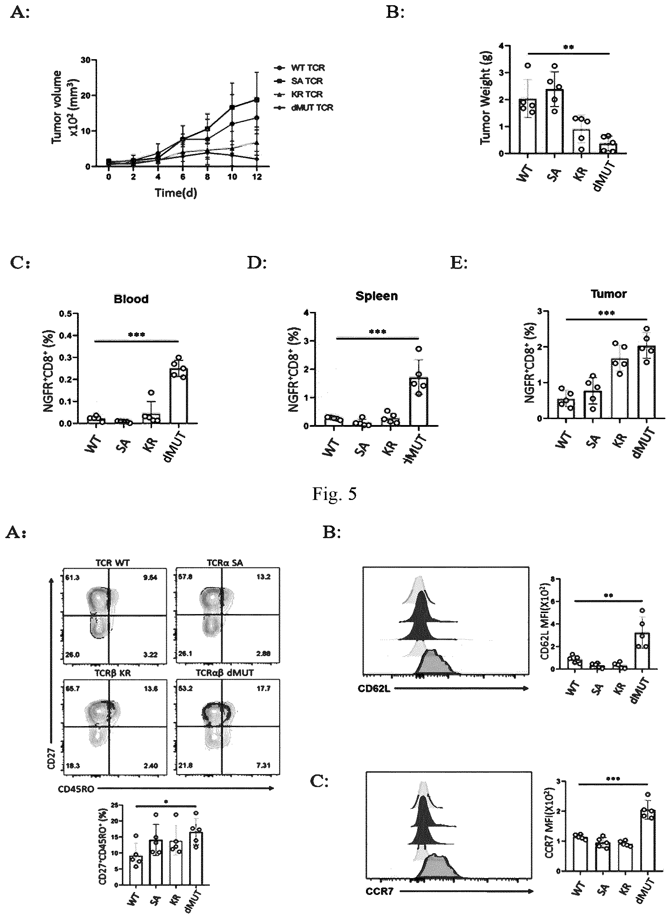

FIG. 5. is a comparison chart of anti-tumor effects of 1G4-TCR-T cells with different mutations. (A) Statistical chart of tumor size at different times in K562-NYESO-1 NSG tumor-bearing mice inoculated with 1G4-TCR-T cells. On the twelfth day after the adoptive transfer of T cells in K562-NYESO-1 NSG tumor-bearing mice, detecting the weight of tumor tissues in different mutation groups (B), and detecting the proportion of adopted TCR-T cells in the blood (C), spleens (D), and tumor tissues (E) of tumor-bearing mice.

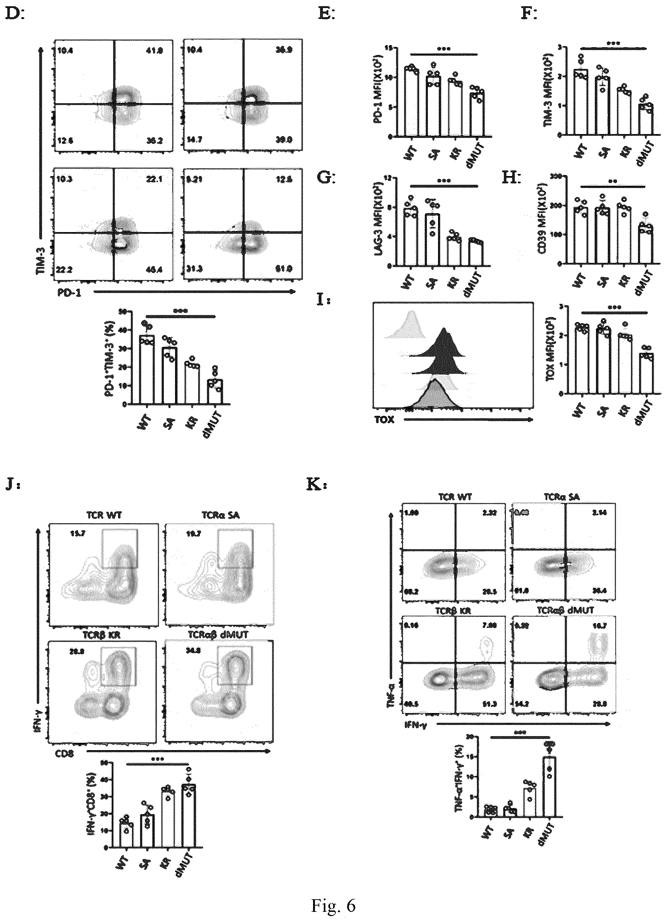

FIG. 6. shows effect of mutations on the function of 1G4-TCR-T cells in mice. (A) In the spleen of NSG tumor-bearing mice, CD45RO and CD27 are used as differentiation indicators for memory T cells, and flow cytometry is performed on different mutation groups. (B) Detecting the expression of memory T cell differentiation indicator CD62L in the tumor tissues of NSG tumor-bearing mice in different mutation groups. (C) Detecting the expression of memory T cell differentiation indicator CCR7 in the tumor tissues of NSG tumor-bearing mice in different mutation groups. (D) Detecting the proportion of PD-1⁺TIM-3⁺ double-positive cells in exhausted T cells in different mutation groups in tumor tissues of NSG tumor-bearing mice and drawing statistical charts. Detecting the expression of immunosuppressive molecules of TCR-T cells, such as PD-1 (E), TIM-3 (F), LAG-3 (G), and CD39 (H), in different mutation groups within tumor tissues of NSG tumor-bearing mice. (I) In tumor tissues of NSG tumor-bearing mice, detecting the expression of transcription factor TOX in different mutation groups. (J) Detecting the proportion of IFN⁻γ⁺ positive CD8⁺ T cells in tumor tissues of NSG tumor-bearing mice. (K) Detecting the proportion of IFN⁻γ⁺ TNF⁻α⁺ double positive TCR-T cells in tumor tissues of NSG tumor-bearing mice.

### DETAILED DESCRIPTION

On one aspect, the present disclosure provides a TCR α chain or fragment thereof isolated from a T cell receptor (TCR). The TCR α chain includes a TCR α chain constant region. The TCR α chain constant region includes a TCR α chain extracellular constant region, a TCR α chain transmembrane region, and a TCR α chain intracellular constant region that are connected in a sequential order. Optionally, the TCR α chain further includes a TCR α chain variable region.

In some embodiments, the TCR α chain constant region and the corresponding TCR α chain extracellular constant region, the corresponding TCR α chain transmembrane region, and the corresponding TCR α chain intracellular constant region thereof, originate from a wild-type T cell receptor (TCR) constant regions of human, mouse, or other mammalian species respectively.

In some embodiments, the TCR α chain intracellular constant region is a wild-type TCR α chain intracellular constant region of human, mouse, or other mammalian species. For example, the wild-type TCR α chain intracellular constant region includes an amino acid sequence set forth in SEQ ID NO: 3 or consists of the amino acid sequence.

In some embodiments, the TCR α chain intracellular constant region is a mutated TCR α chain intracellular constant region. The mutated TCR α chain intracellular constant region is formed by at least one serine mutated to alanine in a wild-type TCR α chain intracellular constant region. For example, the mutated TCR α chain intracellular constant region is formed by at least one serine mutated to alanine in the wild-type TCR α chain intracellular constant region of human, mouse, or other mammalian species.

In some embodiments, the mutated TCR α chain intracellular constant region is formed by at least one serine mutated to alanine in a wild-type TCR α chain intracellular constant region. The wild-type TCR α chain intracellular constant region includes an amino acid sequence set forth in SEQ ID NO: 3 or consists of the amino acid sequence. For example, the mutated TCR α chain intracellular constant region is formed by one or two (all) serine mutation(s) to alanine(s) in the amino acid sequence set forth in SEQ ID NO: 3.

In some embodiments, the mutated TCR α chain intracellular constant region includes an amino acid sequence set forth in SEQ ID NO: 4 or consists of the amino acid sequence.

In some embodiments, the TCR α chain includes a TCR α chain variable region. The TCR α chain variable region can combine and recognize one or more antigens, including but not limited to polypeptide antigens (such as NYESO-1, AFP, and MART-1), lipid antigens (such as β-GlcCer, eLPA, and LPE) and polysaccharide antigens (such as CA199, CA72-4, and CA125).

In some embodiments, the antigens are tumor antigens, microbial antigens, or autoantigens. For example, one or more antigens of BCMA, CA9, CTAG, CCL-1, CSPG4, EGFR, EPG-2, EPG-40, FCRL5, FBP, OGD2, GPC3, GPRC5D, HER3, HER4, HLA-A1, HLA-A2, LRRC8A, CMV, MUC1, MUC16, MART-1, NCAM, PRAME, PSCA, PSMA, ROR1, TPBG, TAG72, TRP1, TRP2, VEGFR, VEGFR2, WT-1, MAGE-A1/A3/A4/A6/A10/C2, gp100, CEA, NYESO-1, AFP, MART-1, HERV-E, HER2, LMP1/2, BRLF-1, BMLF-1, HPV-16E6/E7, KRAS G12D, KRAS G12V, TP53 R175H, β-GlcCer, eLPA, LPE, CA199, CA72-4 or CA125.

In some embodiments, the antigens are tumor antigens, such as one or more of MAGE-A1/A3/A4/A6/A10/C2, gp100, CEA, NYESO-1, AFP, MART-1, HERV-E, HER2, LMP1/2, BRLF-1, BMLF-1, HPV-16E6/E7, KRAS G12D, KRAS G12V, TP53 R175H, β-GlcCer, eLPA, LPE, CA199, CA72-4 or CA125. The antigens can be cell surface antigens or intracellular antigens.

In some embodiments, the TCR α chain variable region includes or consists of an amino acid sequence set forth in SEQ ID NO: 13, which recognizes a tumor antigen MART-1; or includes or consists of an amino acid sequence set forth in SEQ ID NO: 14, which recognizes a tumor antigen NYESO-1.

In some embodiments, the TCR α chain variable region includes a complementary determining region (CDR), such as CDR1, CDR2, and CDR3. The complementary determining region is included in an amino acid sequence set forth in SEQ ID NO: 13 or in an amino acid sequence set forth in SEQ ID NO: 21.

In some embodiments, the TCR α chain variable region includes a CDR1 of the amino acid sequence set forth in SEQ ID NO: 14, a CDR2 of the amino acid sequence set forth in SEQ ID NO: 15, and a CDR3 of the amino acid sequence set forth in SEQ ID NO: 16, which recognizes a tumor antigen MART-1; or includes a CDR1 of the amino acid sequence set forth in SEQ ID NO: 22, a CDR2 of the amino acid sequence set forth in SEQ ID NO: 23 and a CDR3 of the amino acid sequence set forth in SEQ ID NO: 24, which recognizes a tumor antigen NYESO-1.

In some embodiments, the TCR α chain extracellular constant region is a TCR α chain extracellular constant region derived from human, mouse, or other mammalian species.

In some embodiments, the TCR α chain extracellular constant region is an amino acid sequence including SEQ ID NO: 1, or includes an amino acid sequence with 1, 2, 3, 4, 5, 6, 7, 8, 9, or 10 mutations compared to the amino acid sequence set forth in SEQ ID NO: 1, or includes an amino acid sequence with at least 90%, 91%, 92%, 93%, 94%, 95%, 96%, 97%, 98%, or 99% identity with the amino acid sequences set forth in SEQ ID NO: 1, or consists of the amino acid sequence.

In some embodiments, the TCR α chain transmembrane region is a TCR α chain transmembrane region derived from human, mouse, or other mammalian species.

In some embodiments, the TCR α chain transmembrane region is an amino acid sequence including SEQ ID NO: 2, or includes an amino acid sequence with 1, 2, 3, 4, 5, 6, 7, 8, 9, or 10 mutations compared to the amino acid sequence set forth in SEQ ID NO: 2, or includes an amino acid sequence with at least 90%, 91%, 92%, 93%, 94%, 95%, 96%, 97%, 98%, or 99% homology with the amino acid sequences set forth in SEQ ID NO: 2, or consists of the amino acid sequence.

In some embodiments, the TCR α chain constant region includes an amino acid sequence set forth in SEQ ID NO: 5 or SEQ ID NO: 6 or consists of the amino acid sequence.

On one aspect, the present disclosure provides a TCR β chain or fragment thereof isolated from a T cell receptor (TCR). The TCR β chain includes a TCR β chain constant region. The TCR β chain constant region includes a TCR β chain extracellular constant region, a TCR β chain transmembrane region, and a TCR β chain intracellular constant region that are connected in a sequential order. Optionally, the TCR β chain further includes a TCR β chain variable region.

In some embodiments, the TCR β chain constant region and the corresponding TCR β chain extracellular constant region, the corresponding TCR β chain transmembrane region, and the corresponding TCR β chain intracellular constant region thereof, originate from the wild-type T cell receptor (TCR) constant regions of human, mouse, or other mammalian species respectively.

In some embodiments, the TCR β chain intracellular constant region is a wild-type TCR β chain intracellular constant region of human, mouse, or other mammalian species. For example, the wild-type TCR β chain intracellular constant region includes an amino acid sequence set forth in SEQ ID NO: 9 or consists of the amino acid sequence. For example, the wild-type TCR β chain intracellular constant region includes an amino acid sequence set forth in SEQ ID NO: 47 or consists of the amino acid sequence. For example, the wild-type TCR β chain intracellular constant region includes an amino acid sequence set forth in SEQ ID NO: 48 or consists of the amino acid sequence.

In some embodiments, the TCR β chain intracellular constant region is a mutated TCR β chain intracellular constant region. The mutated TCR β chain intracellular constant region is formed by at least one lysine mutated to arginine or alanine in a wild-type TCR β chain intracellular constant region. For example, the mutated TCR β chain intracellular constant region is formed by at least one lysine mutated to arginine or alanine in the wild-type TCR β chain intracellular constant region of human, mouse, or other mammalian species.

In some embodiments, the mutated TCR β chain intracellular constant region is formed by at least one serine mutated to alanine in a wild-type TCR β chain intracellular constant region. The wild-type TCR β chain intracellular constant region includes an amino acid sequence set forth in SEQ ID NO: 9, SEQ ID NO: 47, or SEQ ID NO: 48, or consists of the amino acid sequence. For example, the mutated TCR β chain intracellular constant region is formed by one, two, or three (all) lysine(s) mutations to arginine(s) or alanine(s) in the amino acid sequence set forth in SEQ ID NO: 9. For example, the mutated TCR β chain intracellular constant region is formed by one or two (all) lysine(s) mutations to arginine(s) or alanine(s) in the amino acid sequence set forth in SEQ ID NO: 47 or SEQ ID NO: 48.

In some embodiments, the mutated TCR β chain intracellular constant region includes an amino acid sequence set forth in SEQ ID NO: 10 or consists of the amino acid sequence.

In some embodiments, the TCR β chain includes a TCR β chain variable region. The TCR β chain variable region can combine and recognize one or more antigens, including but not limited to polypeptide antigens (such as NYESO-1, AFP, and MART-1), lipid antigens (such as β-GlcCer, eLPA, and LPE) and polysaccharide antigens (such as CA199, CA72-4, and CA125).

In some embodiments, the antigens are tumor antigens, microbial antigens, or autoantigens. For example, one or more antigens of BCMA, CA9, CTAG, CCL-1, CSPG4, EGFR, EPG-2, EPG-40, FCRL5, FBP, OGD2, GPC3, GPRC5D, HER3, HER4, HLA-A1, HLA-A2, LRRC8A, CMV, MUC1, MUC16, MART-1, NCAM, PRAME, PSCA, PSMA, ROR1, TPBG, TAG72, TRP1, TRP2, VEGFR, VEGFR2, WT-1, MAGE-A1/A3/A4/A6/A10/C2, gp100, CEA, NYESO-1, AFP, MART-1, HERV-E, HER2, LMP1/2, BRLF-1, BMLF-1, HPV-16E6/E7, KRAS G12D, KRAS G12V, TP53 R175H, β-GlcCer, eLPA, LPE, CA199, CA72-4 or CA125.

In some embodiments, the antigens are tumor antigens, such as one or more of MAGE-A1/A3/A4/A6/A10/C2, gp100, CEA, NYESO-1, AFP, MART-1, HERV-E, HER2, LMP1/2, BRLF-1, BMLF-1, HPV-16E6/E7, KRAS G12D, KRAS G12V, TP53 R175H, β-GlcCer, eLPA, LPE, CA199, CA72-4 or CA125. The antigens can be cell surface antigens or intracellular antigens.

In some embodiments, the TCR β chain variable region includes or consists of an amino acid sequence set forth in SEQ ID NO: 17, which recognizes a tumor antigen MART-1; or includes or consists of an amino acid sequence set forth in SEQ ID NO: 25, which recognizes a tumor antigen NYESO-1.

In some embodiments, the TCR β chain variable region includes a complementary determining region (CDR), such as CDR1, CDR2, and CDR3. The complementary determining region is included in an amino acid sequence set forth in SEQ ID NO: 17 or in an amino acid sequence set forth in SEQ ID NO: 25.

In some embodiments, the TCR β chain variable region includes a CDR1 of the amino acid sequence set forth in SEQ ID NO: 18, a CDR2 of the amino acid sequence set forth in SEQ ID NO: 19, and a CDR3 of the amino acid sequence set forth in SEQ ID NO: 20, which recognizes a tumor antigen MART-1; or includes a CDR1 of the amino acid sequence set forth in SEQ ID NO: 26, a CDR2 of the amino acid sequence set forth in SEQ ID NO: 27 and a CDR3 of the amino acid sequence set forth in SEQ ID NO: 28, which recognizes a tumor antigen NYESO-1.

In some embodiments, the TCR β chain extracellular constant region is a TCR β chain extracellular constant region derived from human, mouse, or other mammalian species.

In some embodiments, the TCR β chain extracellular constant region is an amino acid sequence including SEQ ID NO: 7, or includes an amino acid sequence with 1, 2, 3, 4, 5, 6, 7, 8, 9, or 10 mutations compared to the amino acid sequence set forth in SEQ ID NO: 7, or includes an amino acid sequence with at least 90%, 91%, 92%, 93%, 94%, 95%, 96%, 97%, 98%, or 99% homology with the amino acid sequences set forth in SEQ ID NO: 7, or consists of the amino acid sequence.

In some embodiments, the TCR β chain transmembrane region is a TCR β chain transmembrane region derived from human, mouse, or other mammalian species.

In some embodiments, the TCR β chain transmembrane region is an amino acid sequence including SEQ ID NO: 8, or includes an amino acid sequence with 1, 2, 3, 4, 5, 6, 7, 8, 9, or 10 mutations compared to the amino acid sequence set forth in SEQ ID NO: 8, or includes an amino acid sequence with at least 90%, 91%, 92%, 93%, 94%, 95%, 96%, 97%, 98%, or 99% homology with the amino acid sequences set forth in SEQ ID NO: 8, or consists of the amino acid sequence.

In some embodiments, the TCR β chain constant region includes an amino acid sequence set forth in SEQ ID NO: 11 or SEQ ID NO: 12 or consists of the amino acid sequence.

On the other aspect, the present disclosure provides an isolated T cell receptor or fragment thereof. The T cell receptor includes any of the aforementioned TCR α chain and/or TCR β chain.

In some embodiments, the T cell receptor includes any of the aforementioned TCR α chain, wherein the TCR α chain intracellular constant region is selected from:
(1) a wild-type TCR α chain intracellular constant region derived from human, mouse, or other mammalian species;
(2) a wild-type TCR α chain intracellular constant region including an amino acid sequence set forth in SEQ ID NO: 3 or consisting of the amino acid sequence;
(3) a mutated TCR α chain intracellular constant region, where the mutated TCR α chain intracellular constant region is formed by at least one serine mutated to alanine in the wild-type TCR α chain intracellular constant region as described in (1) or (2); or
(4) a mutated TCR α chain intracellular constant region including an amino acid sequence set forth in SEQ ID NO: 4 or consisting of the amino acid sequence.

In some embodiments, the T cell receptor includes any of the aforementioned TCR β chain, wherein the TCR β chain intracellular constant region is selected from:
(1) a wild-type TCR β chain intracellular constant region derived from human, mouse, or other mammalian species;
(2) a wild-type TCR β chain intracellular constant region including an amino acid sequence set forth in SEQ ID NO: 9, SEQ ID NO: 47, or SEQ ID NO: 48, or consisting of the amino acid sequence;
(3) a mutated TCR β chain intracellular constant region, where the mutated TCR β chain intracellular constant region is formed by at least one lysine mutated to arginine or alanine in the wild-type TCR β chain intracellular constant region as described in (1) or (2); or
(4) a mutated TCR β chain intracellular constant region including an amino acid sequence set forth in SEQ ID NO: 10 or consisting of the amino acid sequence.

In some embodiments, the T cell receptor includes any one of the aforementioned TCR α chain and TCR β chain, wherein the TCR α chain intracellular constant region and the TCR β chain intracellular constant region are not both wild-type. For example, when the TCR α chain intracellular constant region is any one of the aforementioned wild-type TCR α chain intracellular constant regions, the TCR β chain intracellular constant region is any one of the aforementioned mutated TCR β chain intracellular constant regions. When the TCR α chain intracellular constant region is any one of the aforementioned mutated TCR α chain intracellular constant regions, the TCR β chain intracellular constant region is any one of the aforementioned wild-type TCR β chain intracellular constant regions.

In some embodiments, the present disclosure provides an isolated T cell receptor or fragment thereof. The T cell receptor includes the TCR α chain and the TCR β chain. The TCR β chain includes a TCR β chain variable region and a TCR β chain constant region. The TCR β chain constant region includes a TCR β chain extracellular constant region, a TCR β chain transmembrane region, and a TCR β chain intracellular constant region that are connected in a sequential order. The TCR β chain intracellular constant region selected from:
(1) a mutated TCR β chain intracellular constant region, where the mutated TCR β chain intracellular constant region is formed by at least one lysine mutated to arginine or alanine in a wild-type TCR β chain intracellular constant region; or
(2) a mutated TCR β chain intracellular constant region including an amino acid sequence set forth in SEQ ID NO: 10 or consists of the amino acid sequence.

Furthermore, the wild-type TCR β chain intracellular constant region is selected from any of the aforementioned wild-type TCR β chain intracellular constant regions in the present disclosure, such as:
(1) a wild-type TCR β chain intracellular constant region derived from human, mouse, or other mammalian species;
(2) a wild-type TCR β chain intracellular constant region includes an amino acid sequence set forth in SEQ ID NO: 9, SEQ ID NO: 47, or SEQ ID NO: 48, or consists of the amino acid sequence.

Furthermore, the TCR α chain includes a TCR α chain constant region. The TCR α chain constant region includes a TCR α chain extracellular constant region, a TCR α chain transmembrane region, and a TCR α chain intracellular constant region that are connected in a sequential order. The TCR α chain intracellular constant region is selected from any of the wild-type TCR α chain intracellular constant regions or mutated TCR α chain intracellular constant regions aforementioned in the present disclosure, such as:
(1) a wild-type TCR α chain intracellular constant region derived from human, mouse, or other mammalian species;
(2) a wild-type TCR α chain intracellular constant region includes an amino acid sequence set forth in SEQ ID NO: 3 or consists of the amino acid sequence;
(3) a mutated TCR α chain intracellular constant region, where the mutated TCR α chain intracellular constant region is formed by at least one serine mutated to alanine in the wild-type TCR α chain intracellular constant region as described in (1) or (2); or
(4) a mutated TCR α chain intracellular constant region includes an amino acid sequence set forth in SEQ ID NO: 4 or consists of the amino acid sequence.

Furthermore, the TCR α chain includes a TCR α chain variable region.

In some embodiments, the present disclosure provides an isolated T cell receptor or fragment thereof. The T cell receptor includes the TCR α chain and the TCR β chain. The TCR β chain includes a TCR β chain variable region and a TCR β chain constant region. The TCR α chain constant region includes a TCR α chain extracellular constant region, a TCR α chain transmembrane region, and a TCR α chain intracellular constant region that are connected in a sequential order. The TCR α chain intracellular constant region selected from:
(1) a mutated TCR α chain intracellular constant region, where the mutated TCR α chain intracellular constant region is formed by at least one serine mutated to alanine in the wild-type TCR α chain intracellular constant region; or
(2) a mutated TCR α chain intracellular constant region including an amino acid sequence set forth in SEQ ID NO: 4 or consisting of the amino acid sequence.

Furthermore, the wild-type TCR α chain intracellular constant region is selected from any of the aforementioned wild-type TCR α chain intracellular constant regions in the present disclosure, such as:
(1) a wild-type TCR α chain intracellular constant region derived from human, mouse, or other mammalian species;
(2) a wild-type TCR α chain intracellular constant region includes an amino acid sequence set forth in SEQ ID NO: 3 or consists of the amino acid sequence.

Furthermore, the TCR β chain includes a TCR β chain constant region. The TCR β chain constant region includes a TCR β chain extracellular constant region, a TCR β chain transmembrane region, and a TCR β chain intracellular constant region that are connected in a sequential order. The TCR β chain intracellular constant region is selected from any of the wild-type TCR β chain intracellular constant regions or mutated TCR β chain intracellular constant regions aforementioned in the present disclosure, such as:
(1) a wild-type TCR β chain intracellular constant region derived from human, mouse, or other mammalian species;
(2) a wild-type TCR β chain intracellular constant region includes an amino acid sequence set forth in SEQ ID NO: 9, SEQ ID NO: 47, or SEQ ID NO: 48, or consists of the amino acid sequence.
(3) a mutated TCR β chain intracellular constant region, where the mutated TCR β chain intracellular constant region is formed by at least one lysine mutated to arginine or alanine in the wild-type TCR β chain intracellular constant region as described in (1) or (2); or
(4) a mutated TCR β chain intracellular constant region includes an amino acid sequence set forth in SEQ ID NO: 10 or consists of the amino acid sequence.

Furthermore, the TCR β chain includes a TCR β chain variable region.

In some embodiments, the T cell receptor includes the wild-type TCR α chain intracellular constant region includes the amino acid sequence set forth in SEQ ID NO: 3 or consists of the amino acid sequence; and/or includes the mutated TCR β chain intracellular constant region includes the amino acid sequence set forth in SEQ ID NO: 10 or composed of the amino acid sequence.

In some embodiments, the T cell receptor includes the mutated TCR α chain intracellular constant region includes the amino acid sequence set forth in SEQ ID NO: 4 or composed of the amino acid sequence; and/or includes the wild-type TCR β chain intracellular constant region includes the amino acid sequence set forth in SEQ ID NO: 9, SEQ ID NO: 47, or SEQ ID NO: 48, or composed of the amino acid sequence.

In some embodiments, the T cell receptor includes the mutated TCR α chain intracellular constant region includes the amino acid sequence set forth in SEQ ID NO: 4 or consists of the amino acid sequence; and/or includes the mutated TCR β chain intracellular constant region includes the amino acid sequence set forth in SEQ ID NO: 10 or composed of the amino acid sequence.

In some embodiments, any of the aforementioned T cell receptor may include any of the aforementioned TCR α chain variable regions and/or TCR β chain variable regions. The TCR α chain variable regions and/or TCR β chain variable region can combine and recognize one or more antigens, including but not limited to polypeptide antigens (such as NYESO-1, AFP, and MART-1), lipid antigens (such as β-GlcCer, eLPA, and LPE) and polysaccharide antigens (such as CA199, CA72-4, and CA125). The antigens can be tumor antigens, microbial antigens, or autoantigens. For example, one or more antigens of BCMA, CA9, CTAG, CCL-1, CSPG4, EGFRm, EPG-2, EPG-40, FCRL5, FBP, OGD2, GPC3, GPRC5D, HER3, HER4, HLA-A1, HLA-A2, LRRC8A, CMV, MUC1, MUC16, MART-1, NCAM, PRAME, PSCA, PSMA, ROR1, TPBG, TAG72, TRP1, TRP2, VEGFR, VEGFR2, WT-1, MAGE-A1/A3/A4/A6/A10/C2, gp100, CEA, NYESO-1, AFP, MART-1, HERV-E, HER2, LMP1/2, BRLF-1, BMLF-1, HPV-16E6/E7, KRAS G12D, KRAS G12V, TP53 R175H, β-GlcCer, eLPA, LPE, CA199, CA72-4, or CA125. Preferably, one or more in MAGE-A1/A3/A4/A6/A10/C2, gp100, CEA, NYESO-1, AFP, MART-1, HERV-E, HER2, LMP1/2, BRLF-1, BMLF-1, HPV-16E6/E7, KRAS G12D, KRAS G12V, TP53 R175H, β-GlcCer, eLPA, LPE, CA199, CA72-4, or CA125. The antigens can be cell surface antigens or intracellular antigens.

In some embodiments, any of the aforementioned T cell receptor include any of the aforementioned TCR β chain variable regions, such as:
(1) the TCR β chain variable region includes three complementarity determining regions (CDR) included in an amino acid sequence set forth in SEQ ID NO: 17;
(2) the TCR β chain variable region includes three complementarity determining regions (CDR) included in an amino acid sequence set forth in SEQ ID NO: 25;
(3) the TCR β chain variable region includes CDR1 of an amino acid sequence set forth in SEQ ID NO: 18, CDR2 of an amino acid sequence set forth in SEQ ID NO: 19, and/or CDR3 of an amino acid sequence set forth in SEQ ID NO: 20;
(4) the TCR β chain variable region includes CDR1 of an amino acid sequence set forth in SEQ ID NO: 26, CDR2 of an amino acid sequence set forth in SEQ ID NO: 27, and/or CDR3 of an amino acid sequence set forth in SEQ ID NO: 28;
(5) the TCR β chain variable region includes an amino acid sequence set forth in SEQ ID NO: 17 or consists of the amino acid sequence; or
(6) the TCR β chain variable region includes an amino acid sequence set forth in SEQ ID NO: 25 or consists of the amino acid sequence.

In some embodiments, any of the aforementioned T cell receptor include any of the aforementioned TCR α chain variable regions, such as:
(1) the TCR α chain variable region includes three complementarity determining regions (CDR) included in an amino acid sequence set forth in SEQ ID NO: 13;
(2) the TCR α chain variable region includes three complementarity determining regions (CDR) included in an amino acid sequence set forth in SEQ ID NO: 21;
(3) the TCR α chain variable region includes CDR1 of an amino acid sequence set forth in SEQ ID NO: 14, CDR2 of an amino acid sequence set forth in SEQ ID NO: 15, and/or CDR3 of an amino acid sequence set forth in SEQ ID NO: 16;
(4) the TCR α chain variable region includes CDR1 of an amino acid sequence set forth in SEQ ID NO: 22, CDR2 of an amino acid sequence set forth in SEQ ID NO: 23, and/or CDR3 of an amino acid sequence set forth in SEQ ID NO: 24;
(5) the TCR α chain variable region includes an amino acid sequence set forth in SEQ ID NO: 13 or consists of the amino acid sequence; or
(6) the TCR α chain variable region includes an amino acid sequence set forth in SEQ ID NO: 21 or consists of the amino acid sequence.

In some embodiments, the TCR α chain variable regions and TCR β chain variable regions of any of the aforementioned T cell receptor are selected as:
(1) the TCR α chain variable region including three complementarity determining regions (CDR) included in an amino acid sequence set forth in SEQ ID NO: 13, and the TCR β chain variable region including three complementarity determining regions (CDR) included in an amino acid sequence set forth in SEQ ID NO: 17;
(2) the TCR α chain variable region including three complementarity determining regions (CDR) included in an amino acid sequence set forth in SEQ ID NO: 21, and the TCR β chain variable region including three complementarity determining regions (CDR) included in an amino acid sequence set forth in SEQ ID NO: 25;
(3) the TCR α chain variable region including CDR1 of an amino acid sequence set forth in SEQ ID NO: 14, CDR2 of an amino acid sequence set forth in SEQ ID NO: 15, and CDR3 of an amino acid sequence set forth in SEQ ID NO: 16, and the TCR β chain variable region including CDR1 of an amino acid sequence set forth in SEQ ID NO: 18, CDR2 of an amino acid sequence set forth in SEQ ID NO: 19, and CDR3 of an amino acid sequence set forth in SEQ ID NO: 20;
(4) the TCR α chain variable region including CDR1 of an amino acid sequence set forth in SEQ ID NO: 22, CDR2 of an amino acid sequence set forth in SEQ ID NO: 23, and CDR3 of an amino acid sequence set forth in SEQ ID NO: 24, and the TCR β chain variable region including CDR1 of an amino acid sequence set forth in SEQ ID NO: 26, CDR2 of an amino acid sequence set forth in SEQ ID NO: 27, and CDR3 of an amino acid sequence set forth in SEQ ID NO: 28;
(5) the TCR α chain variable region including an amino acid sequence set forth in SEQ ID NO: 13 or consisting of the amino acid sequence, and the TCR β chain variable region including an amino acid sequence set forth in SEQ ID NO: 17 or consisting of the amino acid sequence; or
(6) the TCR α chain variable region including an amino acid sequence set forth in SEQ ID NO: 21 or consisting of the amino acid sequence, and the TCR β chain variable region including an amino acid sequence set forth in SEQ ID NO: 25 or consisting of the amino acid sequence.

In some embodiments, the T cell receptor includes any of the aforementioned TCR α chain extracellular constant regions and/or TCR β chain extracellular constant regions.

In some embodiments, the T cell receptor includes any of the aforementioned TCR α chain transmembrane regions and/or TCR β chain transmembrane regions.

In some embodiments, the T cell receptor includes any of the aforementioned TCR α chain constant regions and/or TCR β chain constant regions. For example, the TCR α chain constant region and the TCR β chain constant region are selected from:
(1) the TCR α chain constant region including an amino acid sequence set forth in SEQ ID NO: 5 or consisting of the amino acid sequence, and the TCR β chain constant region including an amino acid sequence set forth in SEQ ID NO: 12 or consisting of the amino acid sequence;
(2) the TCR α chain constant region including an amino acid sequence set forth in SEQ ID NO: 6 or consisting of the amino acid sequence, and the TCR β chain constant region including an amino acid sequence set forth in SEQ ID NO: 12 or consisting of the amino acid sequence; or
(3) the TCR α chain constant region including an amino acid sequence set forth in SEQ ID NO: 6 or consisting of the amino acid sequence, and the TCR β chain constant region including an amino acid sequence set forth in SEQ ID NO: 11 or consisting of the amino acid sequence.

On the other aspect, the present disclosure provides one or more T cell receptors F5-TCR, including F5-WT TCR, F5-SA TCR, F5-KR TCR, or F5-dMUT TCR. The F5-TCR variable region recognizes tumor antigen MART-1, the F5-TCR α chain variable region is the amino acid sequence of SEQ ID NO: 13, and the F5-TCR β chain variable region is the amino acid sequence of SEQ ID NO: 17.

Furthermore, the F5-WT TCR α chain constant region is the amino acid sequence of SEQ ID NO: 5, and the F5-WT TCR β chain constant region is the amino acid sequence of SEQ ID NO: 11.

Furthermore, the F5-SA TCR α chain constant region is the amino acid sequence of SEQ ID NO: 6, and the F5-SA TCR β chain constant region is the amino acid sequence of SEQ ID NO: 11.

Furthermore, the F5-KR TCR α chain constant region is the amino acid sequence of SEQ ID NO: 5, and the F5-KR TCR β chain constant region is the amino acid sequence of SEQ ID NO: 12.

Furthermore, the F5-dMUT TCR α chain constant region is the amino acid sequence of SEQ ID NO: 6, and the F5-dMUT TCR β chain constant region is the amino acid sequence of SEQ ID NO: 12.

On the other aspect, the present disclosure provides one or more T cell receptors 1G4-TCR, including 1G4-WT TCR, 1G4-SA TCR, 1G4-KR TCR, or 1G4-dMUT TCR. The 1G4-TCR variable region recognizes tumor antigen NYESO-1, the1G4-TCR α chain variable region is the amino acid sequence of SEQ ID NO: 21, and the 1G4-TCR β chain variable region is the amino acid sequence of SEQ ID NO: 25.

Furthermore, the 1G4-WT TCR α chain constant region is the amino acid sequence of SEQ ID NO: 5, and the 1G4-WT TCR β chain constant region is the amino acid sequence of SEQ ID NO: 11.

Furthermore, the 1G4-SA TCR α chain constant region is the amino acid sequence of SEQ ID NO: 6, and the 1G4-SA TCR β chain constant region is the amino acid sequence of SEQ ID NO: 11.

Furthermore, the 1G4-KR TCR α chain constant region is the amino acid sequence of SEQ ID NO: 5, and the 1G4-KR TCR β chain constant region is the amino acid sequence of SEQ ID NO: 12.

Furthermore, the 1G4-dMUT TCR α chain constant region is the amino acid sequence of SEQ ID NO: 6, and the 1G4-dMUT TCR β chain constant region is the amino acid sequence of SEQ ID NO: 12.

On the other aspect, any of the aforementioned TCR α chain, TCR β chain, or T cell receptor further binds to a signal peptide, which forms a signal peptide-variable region structure with the TCR α chain variable region and/or TCR β chain variable region.

In some embodiments, the signal peptide is selected from a human growth hormone signal peptide, a CD8α signal peptide, and an immunoglobulin signal peptide.

In some embodiments, the signal peptide includes or consists of an amino acid sequence set forth in SEQ ID NO: 29 and/or SEQ ID NO: 30.

In some embodiments, an amino acid sequence of TCR α chain signal peptide includes or consists of the amino acid sequence set forth in SEQ ID NO: 29.

In some embodiments, an amino acid sequence of TCR β chain signal peptide includes or consists of the amino acid sequence set forth in SEQ ID NO: 30.

Any of the aforementioned parts that form any of the TCR α chain, TCR β chain, or T cell receptor aforementioned in the present disclosure can be directly connected to each other or connected by a linker sequence. The linker sequence can be a sequence of adjacent such as GGGS, GGGGS, GSGSA, GGSGG and other motifs, repeating 1-5 motifs, and with a length of 3-25 amino acid residues.

On the other aspect, the present disclosure provides an isolated nucleic acid or fragment thereof, which encodes any of the aforementioned isolated TCR α chain or fragment thereof, TCR β chain or fragment thereof, or T cell receptor or fragment thereof.

In some embodiments, the nucleic acid sequence encoding the wild-type TCR α chain constant region includes or consists of a nucleic acid sequence of SEQ ID NO: 31.

In some embodiments, the nucleic acid sequence encoding the wild-type TCR β chain constant region includes or consists of a nucleic acid sequence of SEQ ID NO: 32.

In some embodiments, the nucleic acid sequence encoding the SA TCR α chain constant region includes or consists of a nucleic acid sequence of SEQ ID NO: 33.

In some embodiments, the nucleic acid sequence encoding the KR TCR β chain constant region includes or consists of a nucleic acid sequence of SEQ ID NO: 34.

In some embodiments, the nucleic acid sequence encoding the F5-TCR α chain variable region includes or consists of a nucleic acid sequence of SEQ ID NO: 35.

In some embodiments, the nucleic acid sequence encoding the F5-TCR β chain variable region includes or consists of a nucleic acid sequence of SEQ ID NO: 36.

In some embodiments, the nucleic acid sequence encoding the 1G4-TCR α chain variable region includes or consists of a nucleic acid sequence of SEQ ID NO: 37.

In some embodiments, the nucleic acid sequence encoding the 1G4-TCR β chain variable region includes or consists of a nucleic acid sequence of SEQ ID NO: 38.

In some embodiments, the nucleic acid sequence encoding F5-WT-TCR includes or consists of a nucleic acid sequence of SEQ ID NO: 39.

In some embodiments, the nucleic acid sequence encoding F5-SA-TCR includes or consists of a nucleic acid sequence of SEQ ID NO: 40.

In some embodiments, the nucleic acid sequence encoding F5-KR-TCR includes or consists of a nucleic acid sequence of SEQ ID NO: 41.

In some embodiments, the nucleic acid sequence encoding F5-dMUT-TCR includes or consists of a nucleic acid sequence of SEQ ID NO: 42.

In some embodiments, the nucleic acid sequence encoding 1G4-WT-TCR includes or consists of a nucleic acid sequence of SEQ ID NO: 43.

In some embodiments, the nucleic acid sequence encoding 1G4-SA-TCR includes or consists of a nucleic acid sequence of SEQ ID NO: 44.

In some embodiments, the nucleic acid sequence encoding 1G4-KR-TCR includes or consists of a nucleic acid sequence of SEQ ID NO: 45.

In some embodiments, the nucleic acid sequence encoding 1G4-dMUT-TCR includes or consists of a nucleic acid sequence of SEQ ID NO: 46. On the other aspect, the present disclosure provides a nucleic acid construct, which includes any of the aforementioned isolated nucleic acids or fragments thereof.

In some embodiments, the nucleic acid construct further includes one or more regulatory sequences operably connected to the aforementioned nucleic acid. For example, including but not limited to promoter sequences that display transcriptional activity in host cells, transcription terminator sequences that are recognized and terminated in host cells and operably connected to the 3 'end of the coding sequence, and leader sequences that are important to host cell translation and operably connected to the 5' end of the coding sequence.

On the other aspect, the present disclosure provides a vector, which includes any of the aforementioned nucleic acid construct.

In some embodiments, the vector is an expression vector or a crisper gene editing vector, such as a retrovirus vector, lentivirus vector, baculovirus vector, herpesvirus vector, adenovirus vector, and adeno-associated virus (AAV) vector.

In some embodiments, the construction of the retrovirus vector mainly includes an origin of replication, 5'-LTR, 3'-LTR, and any of the aforementioned nucleic acid sequences or nucleic acid construct.

Usually, the promoter is operably linked to the nucleic acid sequence encoding T cell receptors, and the nucleic acid construct is incorporated into the expression vector to achieve the expression of the polynucleotide sequence encoding T cell receptors. The nucleic acid sequence encoding the T cell receptor of the present disclosure can be cloned into various types of vectors, for example, including but not limited to plasmids, phages, animal viruses, etc.

An appropriate expression vector includes one or more promoter sequences, origins of replication, appropriate enzyme cutting sites, and optional markers, that play a role in an organism.

An appropriate promoter includes but is not limited to constitutive promoter sequence, such as immediate early cytomegalovirus (CMV) promoter, extended growth factor-1a (EF-1a), simian virus 40 (SV40) early promoter, mouse mammary tumor virus (MMTV), human immunodeficiency virus (HIV), long terminal repeated (LTR) promoter, etc., which can drive high-level expression of any polynucleotide sequence connected to them. The suitable promoter also includes but is not limited to inducible promoters, such as metallothionein promoters and tetracycline promoters, which are used to turn on when expecting the expression of the polynucleotide sequence and off when not expecting the expression.

The optional marker includes marker gene and reporter gene, to facilitate the identification of selectively expressed cells in cell populations infected with viral vectors. Appropriate marker genes include but are not limited to antibiotics resistance genes, Neo genes, etc. Appropriate reporter genes include but are not limited to β-galactosidase, green fluorescent protein gene, luciferase, chloramphenicol acetyltransferase, etc.

On the other aspect, the present disclosure provides an engineered cell, which includes any of the aforementioned nucleic acid constructs or vectors.

In some embodiments, the engineered cell is a primary cell obtained from a subject. In some embodiments, the subject is a mammalian subject, for example, the subject is human.

In some embodiments, the engineered cell is a T cell, preferably a human T cell.

In some implementation methods, the engineered cell is NK cells, NKT cells, macrophages, γδ T cells, human CD4⁺ T cells, CD8⁺ T cells, or a mixed cell population of CD4⁺T/CD8⁺T cells.

Through conventional recombinant DNA technology, the TCR nucleic acid construct or the vector comprising the nucleic acid construct of the present disclosure can be used to express or produce TCR or engineered cells comprising the TCR. Usually, the methods include:
(1) transforming or transducing appropriate host cells by a TCR nucleic acid construct or a vector including the nucleic acid construct;
(2) culturing host cells in appropriate culture media;
(3) harvesting and purifying the engineered cells or TCRs of the present disclosure from the culture media or the cells.

The method of introducing different nucleic acid sequences or vectors into engineered cells is currently well-known in the field, and target genes can be introduced into host cells through physical, chemical, and biological methods.

In some embodiments, the present disclosure uses retroviruses to complete the introduction of target sequences into host cells, and retroviral infection is currently a widely used method for infecting primary cells of humans or mice. In some embodiments of the present disclosure, after different mutated TCR nucleic acid sequences are constructed into retroviral vectors, preferred virus envelope proteins or capsid proteins are used for packaging and producing virus particles. Subsequently, the recombinant virus particles are transmitted into the host cells or *in vitro* cultured cells to complete the expression of the target gene in the host cells.

The aforementioned virus particles include TCR nucleic acid sequences, retroviral vectors, and nucleic acid sequences of packaging proteins.

On the other aspect, the present disclosure also provides a TCR complex, which is generated in T cells by any of the TCR nucleic acid constructs aforementioned in the present disclosure.

On the other aspect, the present disclosure provides functional testing of TCR-T cells during the process of *in vitro* culture, specifically including TCR-T cell memory phenotype testing, TCR-T cell exhaustion phenotype testing *in vitro* exhaustion models, and TCR-T cell mitochondrial phenotype testing. The aforementioned TCR-T cell memory phenotype testing and TCR-T cell mitochondrial phenotype testing are detected by flow cytometry after activation of human primary T cells. The aforementioned mutated TCR-T cell exhaustion testing uses an exhaustion model constructed *in vitro.* Specifically, CD3 antibodies are used to stimulate TCR-T cells with different mutations *in vitro* for multiple rounds, and then flow cytometry is used to detect the expression of exhaustion-related molecules.

On the other aspect, the present disclosure provides the disclosure of any of the aforementioned isolated TCR α chain or fragment thereof, TCR β chain or fragment thereof, T cell receptor or fragment thereof, nucleic acid or fragment thereof, nucleic acid constructs, vectors, or engineered cells in any one or more of the following uses:
(1) the application of inhibiting T cell receptor degradation during the process of antigen stimulation;
(2) providing a killing effect on tumors or inhibiting tumor growth;
(3) maintaining the proliferation ability of T cells;
(4) preparation of anti-tumor immune drugs and cells.

On the other aspect, the mutated TCR-T cell therapy provided in the present disclosure can be used alone or in combination with other therapies, or in combination with PD-1/PD-L1 antibody, or in combination with cytokine therapy, or in combination with radiotherapy and chemotherapy. When the aforementioned therapies are used alone or in combination, the quantity and frequency of use are determined by various factors such as the characteristics of the disease and the severity of the disease. In theory, the TCR-T cell therapy provided in the present disclosure can be used multiple times at low doses, with a dosage range of 10⁴-10⁹ cells/kg. The adoption therapy for TCR-T cells provided in the present disclosure follows internationally recognized adoption techniques. In an embodiment in the present article, 5×10⁶ TCR-T cells were administered via tail vein injection. In principle, the combination of T cell therapy can be directly injected into tumor tissue or infection site.

On the other aspect, the present disclosure provides a modification method for the T cell receptor, comprising:
(1) mutating at least one lysine in the wild-type TCR β chain intracellular constant region to arginine or alanine; and/or
(2) mutating at least one serine in the wild-type TCR α chain intracellular constant region to alanine.

In order to better understand the disclosed content of the present disclosure, certain terms are defined as follows.

T cell receptor (TCR) is a molecule present on the surface of T cells, responsible for recognizing peptide-MHC complexes. In some embodiments, the TCR is a complete or full-length TCR. In some embodiments, the present disclosure provides a TCR fragment that is smaller than the full-length TCR but still retains binding to a specific antigenic peptide of MHC molecules (MHC-peptide complex). In some embodiments, the TCR is a heterodimer composed of α and β chains. In some embodiments, the TCR can also be a single-chain TCR (scTCR).

The term "variable region" or "variable domain" refers to a domain of TCR α or β chain, which is involved in the binding of TCR to antigen-MHC complexes. The variable regions of the natural TCR α chain or β chain typically have similar structures. Each structural region includes four conserved framework regions (FR) and three highly variable regions or complementary determining regions (CDR). The CDR3 in each variable region is the main CDR responsible for recognizing processed antigens. A single TCR α chain variable region or TCR β chain variable region may be sufficient to confer binding to peptide-MHC complexes.

It should be understood that in some embodiments, the TCR α chain of the present disclosure can be a human TCR α chain, a humanized TCR α chain, a chimeric TCR α chain, or a murine TCR α chain. In some embodiments of the present disclosure, the TCR α chain is a chimeric TCR α chain that includes sequences derived from more than one species, such as sequences derived from humans and mice. For example, exchanging human TCR constant regions with mouse counterparts can improve the function and expression levels of human T cells (see, for example, Daniel Sommermeyer et al., J Immunol. 2010 Jun 1; 184(11):6223-31., which is incorporated by reference into this article). Therefore, TCR can include both human-derived variable regions and mouse-derived constant regions.

Similarly, in some embodiments, the TCR β chain of the present disclosure can be a human TCR β chain, a humanized TCR β chain, a chimeric TCR β chain, or a murine TCR β chain. The chimeric TCR β chain that includes sequences derived from more than one species, such as sequences derived from humans and mice.

The TCR α chain constant zone and/or TCR β chain constant region described in the present disclosure includes an extracellular constant region, a transmembrane region, and an intracellular constant region that are connected in a sequential order. The extracellular constant region can include TCR α chain and TCR β chain hinge region, participating in the formation of disulfide bonds of TCR α chains TCR β chains. The transmembrane region is also a constant region, and its role includes participating in the cell membrane anchoring of the TCR α chain and the TCR β chain and forming the TCR-CD3 complex by its interaction with CD3 subunits. The possible roles of the intracellular constant region include the conformational transformation and signal transduction of the TCR-CD3 complex after participating in TCR signal transduction.

The term "antigen" refers to molecules on the cell surface or presented by MHC molecules or MHC-like molecules within the cell, and can be bound by antibodies or T cell receptors (TCRs), including but not limited to polypeptide antigens (such as NYESO-1, AFP, and MART-1), lipid antigens (such as β-GlcCer, eLPA, and LPE), and polysaccharide antigens (such as CA199, CA72-4, and CA125). The antigen can be a tumor antigen, such as tumor-associated antigen (TAA) or tumor specific antigen (TSA).

The term "isolated" refers to materials that have been removed from their natural state or artificially manipulated in other ways, such as α chains, β chains, T cell receptors, and nucleic acids as described in this article. Isolated materials can have essentially no components that typically accompany them in their natural state, or can be manipulated to be in an artificial state, together with components that typically accompany them in their natural state. The isolated materials can be in the form of natural, chemically synthesized, or recombinant. The isolated materials can also be or alternatively be in the form of enriched, partially purified, or purified.

The term "wild-type" in the present disclosure refers to a class of naturally occurring amino acid sequences or coding nucleic acid sequences that have at least 90%-100% homology compared to naturally occurring amino acid sequences or coding nucleic acid sequences, or where the amino acid mutations are not more than 1-10 or 1-5 (especially conservative amino acid substitutions) compared to naturally occurring amino acid sequences or coding nucleic acid sequences, and still have the same or similar activity and/or function as the region.

"Wild type" is introduced as a template for the corresponding mutation or mutation combination in the present disclosure, for example, "wild type derived from human, mouse, or other mammalian species" refers to the amino acid sequence or coding nucleic acid sequence of the region with natural humans, natural mice or rats, or other mammalian species, or is composed of the amino acid sequence or coding nucleic acid sequence. The region originating from humans, mice, or other mammalian species also includes such a region, which is basically the same as the amino acid sequence or coding nucleic acid sequence of the region in natural humans, natural mice or rats, or other mammalian species. For example, it has at least 90%-100% homology compared to naturally occurring amino acid sequences or coding nucleic acid sequences, or the amino acid mutation is not more than 1-10 or 1-5 (especially conservative amino acid substitutions) compared to naturally occurring amino acid sequences or coding nucleic acid sequences, and still have the same or similar activity and/or function as the region.

The term "mutation" can be understood as the substitution, deletion, or addition of one or more amino acids or nucleic acids. For example, it can be a conservative substitution of amino acid. The conservative amino acid substitution is known in the art and includes amino acid substitution which one amino acid with certain physical and/or chemical properties is exchanged by another amino acid with the same chemical or physical properties. For example, the conservative amino acid substitution can be an acidic amino acid replacing another acidic amino acid (such as Asp or Glu), an amino acid with a non-polar side chain replacing another amino acid with a non-polar side chain (such as Ala, Gly, Val, He, Leu, Met, Phe, Pro, Trp, Val, etc.), an alkaline amino acid replacing another alkaline amino acid (Lys, Arg, etc.), an amino acid with a polar side chain replacing another amino acid with a polar side chain (Asn, Cys, Gin, Ser, Thr, Tyr, etc.), etc. The conservative substitution can proceed based on, for example, polarity, charge, solubility, hydrophobicity, hydrophilicity, and/or the similarity of the amphiphilic properties of the residues involved.

When involving mutations in the TCR α chain intracellular constant region and/or TCR β chain intracellular constant region of the present disclosure, it is preferred to be the substitution of amino acids. For example, the mutated TCR α chain intracellular constant region refers to the wild-type TCR α chain intracellular constant region with at least one serine replaced by alanine; the mutated TCR β chain intracellular constant region refers to the wild-type TCR β chain intracellular constant region with at least one lysine replaced by arginine or alanine.

Calculating the homology between sequences as follows.

To determine the percentage homology between two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, the length of a reference sequence aligned for comparison purposes is at least 30%, at least 40%, at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate the percentage homology between the sequences. In one preferred embodiment, the percentage homology between two amino acid sequences is determined with the Needlema and Wunsch algorithm ((1970)J. Mol. Biol., 48: 444-453; available at http://www.gcg.com) that has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percentage homology between two nucleotide acid sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5.

The percentage homology between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4:11-17) that has been incorporated into the ALIGN program (version 2.0).

Additionally or alternatively, the nucleic acid sequences and protein sequences described herein can be further used as "query sequences" to perform searches against public databases to, e.g., identify other family member sequences or related sequences.

As known in the field, the term "nucleotide" or "nucleic acid" used interchangeably in this article refers to any length of nucleic acid chain, including DNA and RNA. Nucleic acid can be deoxyribonucleic acid, ribonucleic acid, modified nucleic acid or basic group, and/or the analogs thereof, or any substrate that can be incorporated into the chain through DNA or RNA polymerase.

The nucleic acid sequence described in this article can be obtained by designing primers based on the nucleotide sequence disclosed in this article, using the PCR amplification method, or by preparing cDNA libraries for PCR amplification by the skilled in the art.

It should be understood that "wild-type" and "WT" in this disclosure can be used interchangeably; "SA" refers to at least one serine mutated to alanine in the amino acid sequence of the α chain intracellular constant region; "KR" refers to at least one lysine mutated to arginine in the amino acid sequence of the β chain intracellular constant region; "dMUT" refers to the simultaneous presence in the amino acid sequence of the "SA" mutation in α chain intracellular constant region and the "KR" mutation in β chain intracellular constant region.

It should be understood that F5-TCR in the present disclosure refers to a class of TCR that can recognize tumor antigen MART-1, wherein F5-WT TCR, F5-SA TCR, F5-KR TCR, and F5-dMUT TCR have the same TCR α chain variable region and TCR β chain variable region. The F5-WT TCR, F5-SA TCR, F5-KR TCR, and F5-dMUT TCR are classified based on different mutations in the α chain intracellular constant region or β chain intracellular constant region.

Similarly, 1G4-TCR in the present disclosure refers to a class of TCR that can recognize tumor antigen NYESO-1, wherein 1G4-WT TCR, 1G4-SA TCR, 1G4-KR TCR, or 1G4-dMUT TCR have the same TCR α chain variable region and TCR β chain variable region. The 1G4-WT TCR, 1G4-SA TCR, 1G4-KR TCR, or 1G4-dMUT TCR are classified based on different mutations in the α chain intracellular constant region or β chain intracellular constant region.

"Vector" refers to it can deliver one or more interest genes or sequences into host cells and preferably express the genes or sequences in the host cells. Examples of vectors include but are not limited to viral vectors, plasmids, cosmids, or bacteriophage vectors.

The term "host cell" refers to cells that have introduced exogenous nucleic acids, including the filial generation thereof.

It should be understood that in gene cloning operations, appropriate enzyme cutting sites designed will introduce one or more unrelated residues at both ends of the expressed amino acid sequence, without affecting the activity of the target sequence. The enzyme cutting site includes but is not limited to NotI, BamHI, XhoI, etc.

It should be understood that in order to construct fusion proteins, obtain recombinant proteins that are automatically localized on the host cell membrane, or promote the expression of recombinant proteins, it is necessary to add amino acid fragments to the N-terminus, C-terminus, or appropriate regions of the recombinant protein. For example, the amino acid fragment includes but is not limited to signal peptide, lead peptide, 2A peptide, end extension, etc.

It should be understood that in order to purify the constructed recombinant protein, the amino or carboxyl end of the constructed protein includes one or more protein labels. For example, the protein includes but is not limited to FLAG, HA, c-Myc, Poly-His, etc.

It should be understood that the aforementioned examples (embodiments) are exemplary and not intended to encompass all possible embodiments included in the claims. Without departing from the scope of this disclosure, various deformations and changes can also be made based on the aforementioned embodiments. Similarly, the various technical features of the aforementioned examples can also be arbitrarily combined to form other examples of the present disclosure that may not have been clearly described. Therefore, the aforementioned examples only express several embodiments of the present disclosure and do not limit the protection scope of the present disclosure.

The sequence table and annotations of the present disclosure

| SEQ ID NO | sequences | Description of sequences |
|---|---|---|
| 1 | IQNPEPAVYQLKDPRSQDSTLCLFTDFDSQINVPKT MESGTFITDKTVLDMKAMDSKSNGAIAWSNQTSF TCQDIFKETNATYPSSDVPCDATLTEKSFETDMNLN FQN | The amino acid sequence of the TCR α chain extracellular constant region |
| 2 | LSVMGLRILLLKVAGFNLLMTL | The amino acid sequence of the TCR α chain transmembrane region |
| 3 | RLWSS | The amino acid sequence of the wild-type TCR α chain intracellular constant region |
| 4 | RLWAA | The amino acid sequence of the SA TCR α chain intracellular constant region |
| 5 | IQNPEPAVYQLKDPRSQDSTLCLFTDFDSQINVPKT MESGTFITDKTVLDMKAMDSKSNGAIAWSNQTSF TCQDIFKETNATYPSSDVPCDATLTEKSFETDMNLN FQNLSVMGLRILLLKVAGFNLLMTLRLWSS | The amino acid sequence of the wild-type (WT) TCR α chain constant region |
| 6 | IQNPEPAVYQLKDPRSQDSTLCLFTDFDSQINVPKT MESGTFITDKTVLDMKAMDSKSNGAIAWSNQTSF TCQDIFKETNATYPSSDVPCDATLTEKSFETDMNLN | The amino acid sequence of the SA TCR α chain constant region |
| | FQNLSVMGLRILLLKVAGFNLLMTLRLWAA | |
| 7 | LRNVTPPKVSLFEPSKAEIANKQKATLVCLARGFFP DHVELSWWVNGKEVHSGVSTDPQAYKESNYSYC LSSRLRVSATFWHNPRNHFRCQVQFHGLSEEDKWP EGSPKPVTQNISAEAWGRADCGITSASYHQGVLSA T | The amino acid sequence of the TCR β chain extracellular constant region |
| 8 | ILYEILLGKATLYAVLVSGLVL | The amino acid sequence of the TCR β chain transmembrane region |
| 9 | MAMVKKKNS | The amino acid sequence of wild-type (WT) murine TCR β chain intracellular constant region |
| 10 | MAMVRKRNS | The amino acid sequence based on WT murine KR TCR β chain intracellular constant region |
| 11 | LRNVTPPKVSLFEPSKAEIANKQKATLVCLARGFFP DHVELSWWVNGKEVHSGVSTDPQAYKESNYSYC LSSRLRVSATFWHNPRNHFRCQVQFHGLSEEDKWP EGSPKPVTQNISAEAWGRADCGITSASYHQGVLSA TILYEILLGKATLYAVLVSGLVLMAMVKKKNS | The amino acid sequence of wild-type (WT) murine TCR β chain constant region |
| 12 | LRNVTPPKVSLFEPSKAEIANKQKATLVCLARGFFP DHVELSWWVNGKEVHSGVSTDPQAYKESNYSYC LSSRLRVSATFWHNPRNHFRCQVQFHGLSEEDKWP EGSPKPVTQNISAEAWGRADCGITSASYHQGVLSA TILYEILLGKATLYAVLVSGLVLMAMVRKRNS | The amino acid sequence based on WT murine KR TCR β chain constant region |
| 13 | QQKEVEQNSGPLSVPEGAIASLNCTYSDRGSQSFF WYRQYSGKSPELIMFIYSNGDKEDGRFTAQLNKAS QYVSLLIRDSQPSDSATYLCAVNFGGGKLIFGQGTE LSVKPN | The amino acid sequence of the F5-TCR α chain variable region |
| 14 | DRGSQS | The amino acid sequence of the F5-TCR α chain variable region CDR1 |
| 15 | IYSNGD | The amino acid sequence of the F5-TCR α chain variable region CDR2 |
| 16 | AVNFGGGKLI | The amino acid sequence of the F5-TCR α chain variable region CDR3 |
| 17 | GITQAPTSQILAAGRRMTLRCTQDMRHNAMYWY RQDLGLGLRLIHYSNTAGTTGKGEVPDGYSVSRAN TDDFPLTLASAVPSQTSVYFCASSLSFGTEAFFGQG TRLTVVED | The amino acid sequence of the F5-TCR β chain variable region |
| 18 | MRHNA | The amino acid sequence of the F5-TCR β chain variable region CDR1 |
| 19 | SNTAGT | The amino acid sequence of the F5-TCR β chain variable region CDR2 |
| 20 | ASSLSFGTEAF | The amino acid sequence of the F5-TCR β chain variable region CDR3 |
| 21 | KQEVTQIPAALSVPEGENLVLNCSFTDSAIYNLQWF RQDPGKGLTSLLLIQSSQREQTSGRLNASLDKSSGR STLYIAASQPGDSATYLCAVRPTSGGSYIPTFGRGTS LIVHPY | The amino acid sequence of the 1G4-TCR α chain variable region |
| 22 | DSAIYN | The amino acid sequence of the 1G4-TCR α chain variable region CDR1 |
| 23 | IQSSQ | The amino acid sequence of the 1G4-TCR α chain variable region CDR2 |
| 24 | PTSGGSYIPT | The amino acid sequence of the 1G4-TCR α chain variable region CDR3 |
| 25 | GVTQTPKFQVLKTGQSMTLQCAQDMNHEYMSWY RQDPGMGLRLIHYSVGAGITDQGEVPNGYNVSRS TTEDFPLRLLSAAPSQTSVYFCASSYVGNTGELFFG EGSRLTVLED | The amino acid sequence of the 1G4-TCR β chain variable region |
| 26 | MNHEY | The amino acid sequence of the 1G4-TCR β chain variable region CDR1 |
| 27 | GAGI | The amino acid sequence of the 1G4-TCR β chain variable region CDR2 |
| 28 | YVGN | The amino acid sequence of the 1G4-TCR β chain variable region CDR3 |
| 29 | MATGSRTSLLLAFGLLCLPWLQEASA | The amino acid sequence of TCR α chain signal peptide |
| 30 | MATGSRTSLLLAFGLLCLPCLQEGSA | The amino acid sequence of TCR β chain signal peptide |
| 31 | atccagaaccccgagcccgccgtgtaccagctgaaggaccccagaagccag gacagcaccctgtgcctgttcaccgacttcgacagccagatcaacgtgcccaa gaccatggagagcggcaccttcatcaccgacaagaccgtgctggacatgaag gccatggacagcaagagcaacggcgccatcgcctggtccaaccagaccagct tcacatgccaggacatcttcaaggagaccaacgccacctaccccagcagcgac gtgccctgcgacgccaccctgaccgagaagagcttcgagaccgacatgaacc tgaacttccagaacctgagcgtgatgggcctgagaatcctgctgctgaaggtgg ccggcttcaacctgctgatgaccctgaggctgtggagcagc | The nucleotide sequence of the wild-type (WT) TCR α chain constant region |
| 32 | ctgaggaacgtgaccccccccaaggtgtccctgttcgagcccagcaaggccg agatcgccaacaagcagaaggccaccctggtgtgcctggccaggggcttcttc cccgaccacgtggagctgtcttggtgggtgaacggcaaggaggtgcacagcg gcgtgagcaccgacccccaggcctacaaggagagcaactacagctactgcct gagcagcaggctgagagtgagcgccaccttctggcacaaccccaggaaccac ttccgctgtcaggtgcagttccacggcctgagcgaggaggacaagtggcccg agggcagccccaagcccgtgacccagaacatcagcgccgaggcctggggca | The nucleotide sequence of the wild-type (WT) TCR β chain constant region |
| | gagccgactgcggcatcaccagcgccagctaccaccagggcgtgctgtccgc caccatcctgtacgagatcctgctgggcaaggccacactgtacgccgtgctggt gtccggcctggtgctgatggccatggtgaagaagaagaacagc | |
| 33 | atccagaaccccgagcccgccgtgtaccagctgaaggaccccagaagccag gacagcaccctgtgcctgttcaccgacttcgacagccagatcaacgtgcccaa gaccatggagagcggcaccttcatcaccgacaagaccgtgctggacatgaag gccatggacagcaagagcaacggcgccatcgcctggtccaaccagaccagct tcacatgccaggacatcttcaaggagaccaacgccacctaccccagcagcgac gtgccctgcgacgccaccctgaccgagaagagcttcgagaccgacatgaacc tgaacttccagaacctgagcgtgatgggcctgagaatcctgctgctgaaggtgg ccggcttcaacctgctgatgaccctgaggctgtgggccgcc | The nucleotide sequence of the SA TCR α chain constant region |
| 34 | ctgaggaacgtgaccccccccaaggtgtccctgttcgagcccagcaaggccg agatcgccaacaagcagaaggccaccctggtgtgcctggccaggggcttcttc cccgaccacgtggagctgtcttggtgggtgaacggcaaggaggtgcacagcg gcgtgagcaccgacccccaggcctacaaggagagcaactacagctactgcct gagcagcaggctgagagtgagcgccaccttctggcacaaccccaggaaccac ttccgctgtcaggtgcagttccacggcctgagcgaggaggacaagtggcccg agggcagccccaagcccgtgacccagaacatcagcgccgaggcctggggca gagccgactgcggcatcaccagcgccagctaccaccagggcgtgctgtccgc caccatcctgtacgagatcctgctgggcaaggccacactgtacgccgtgctggt gtccggcctggtgctgatggccatggtgaggaagaggaacagc | The nucleotide sequence of the KR TCR β chain constant region |
| 35 | caacagaaggaggtggagcagaattctggacccctcagtgttccagagggag ccattgcctctctcaactgcacttacagtgaccgaggttcccagtccttcttctggt acagacaatattctgggaaaagccctgagttgataatgttcatatactccaatggt gacaaagaagatggaaggtttacagcacagctcaataaagccagccagtatgtt tctctgctcatcagagactcccagcccagtgattcagccacctacctctgtgccgt gaacttcggaggaggaaagcttatcttcggacagggaacggagttatctgtgaa acccaat | The nucleotide sequence of the F5-TCR α chain variable region |
| 36 | gggatcacccaggcaccaacatctcagatcctggcagcaggacggcgcatga cactgagatgtacccaggatatgagacataatgccatgtactggtatagacaag atctaggactggggctaaggctcatccattattcaaatactgcaggtaccactgg caaaggagaagtccctgatggttatagtgtctccagagcaaacacagatgatttc cccctcacgttggcgtctgctgtaccctctcagacatctgtgtacttctgtgccag cagcctaagtttcggcactgaagctttctttggacaaggcaccagactcacagtt gtagaggac | The nucleotide sequence of the F5-TCR β chain variable region |
| 37 | aaacaggaggtgacgcagattcctgcagctctgagtgtcccagaaggagaaaa cttggttctcaactgcagtttcactgatagcgctatttacaacctccagtggtttag gcaggaccctgggaaaggtctcacatctctgttgcttattcagtcaagtcagaga gagcaaacaagtggaagacttaatgcctcgctggataaatcatcaggacgtagt actttatacattgcagcttctcagcctggtgactcagccacctacctctgtgctgtg aggcccacatcaggaggaagctacatacctacatttggaagaggaaccagcct tattgttcatccgtat | The nucleotide sequence of the 1G4-TCR α chain variable region |
| 38 | ggtgtcactcagaccccaaaattccaggtcctgaagacaggacagagcatgac actgcagtgtgcccaggatatgaaccatgaatacatgtcctggtatcgacaaga cccaggcatggggctgaggctgattcattactcagttggtgctggtatcactgac | The nucleotide sequence of the 1G4-TCR β chain variable region |
| | caaggagaagtccccaatggctacaatgtctccagatcaaccacagaggatttc ccgctcaggctgctgtcggctgctccctcccagacatctgtgtacttctgtgcca gcagttacgtcgggaacaccggggagctgttttttggagaaggctctaggctga ccgtactggaggac | |
| 39 | atggcgacgggttcaagaacttccctacttcttgcatttggcctgctttgtttgccgt ggttacaggaagcctcagcacaacagaaggaggtggagcagaattctggacc cctcagtgttccagagggagccattgcctctctcaactgcacttacagtgaccga ggttcccagtccttcttctggtacagacaatattctgggaaaagccctgagttgat aatgttcatatactccaatggtgacaaagaagatggaaggtttacagcacagctc aataaagccagccagtatgtttctctgctcatcagagactcccagcccagtgattc agccacctacctctgtgccgtgaacttcggaggaggaaagcttatcttcggaca gggaacggagttatctgtgaaacccaatatccagaaccccgagcccgccgtgt accagctgaaggaccccagaagccaggacagcaccctgtgcctgttcaccga cttcgacagccagatcaacgtgcccaagaccatggagagcggcaccttcatca ccgacaagaccgtgctggacatgaaggccatggacagcaagagcaacggcg ccatcgcctggtccaaccagaccagcttcacatgccaggacatcttcaaggaga ccaacgccacctaccccagcagcgacgtgccctgcgacgccaccctgaccga gaagagcttcgagaccgacatgaacctgaacttccagaacctgagcgtgatgg gcctgagaatcctgctgctgaaggtggccggcttcaacctgctgatgaccctga ggctgtggagcagcagggcaaaacgttcgggttcgggtgcgccagtaaagca gacattaaactttgatttgctgaaacttgcaggtgatgtagagtcaaatccaggtc caatggcaacagggagccgaacctctctgctccttgctttcgggctcctttgccta ccgtgcctgcaggagggctcggcagggatcacccaggcaccaacatctcaga tcctggcagcaggacggcgcatgacactgagatgtacccaggatatgagacat aatgccatgtactggtatagacaagatctaggactggggctaaggctcatccatt attcaaatactgcaggtaccactggcaaaggagaagtccctgatggttatagtgt ctccagagcaaacacagatgatttccccctcacgttggcgtctgctgtaccctctc agacatctgtgtacttctgtgccagcagcctaagtttcggcactgaagctttctttg gacaaggcaccagactcacagttgtagaggacctgaggaacgtgaccccccc caaggtgtccctgttcgagcccagcaaggccgagatcgccaacaagcagaag gccaccctggtgtgcctggccaggggcttcttccccgaccacgtggagctgtct tggtgggtgaacggcaaggaggtgcacagcggcgtgagcaccgacccccag gcctacaaggagagcaactacagctactgcctgagcagcaggctgagagtga gcgccaccttctggcacaaccccaggaaccacttccgctgtcaggtgcagttcc acggcctgagcgaggaggacaagtggcccgagggcagccccaagcccgtg acccagaacatcagcgccgaggcctggggcagagccgactgcggcatcacc agcgccagctaccaccagggcgtgctgtccgccaccatcctgtacgagatcct gctgggcaaggccacactgtacgccgtgctggtgtccggcctggtgctgatgg ccatggtgaagaagaagaacagctaa | The full-length nucleotide sequence of F5-WT-TCR |
| 40 | atggcgacgggttcaagaacttccctacttcttgcatttggcctgctttgtttgccgt ggttacaggaagcctcagcacaacagaaggaggtggagcagaattctggacc cctcagtgttccagagggagccattgcctctctcaactgcacttacagtgaccga ggttcccagtccttcttctggtacagacaatattctgggaaaagccctgagttgat aatgttcatatactccaatggtgacaaagaagatggaaggtttacagcacagctc aataaagccagccagtatgtttctctgctcatcagagactcccagcccagtgattc | The full-length nucleotide sequence of F5-SA-TCR |
| | agccacctacctctgtgccgtgaacttcggaggaggaaagcttatcttcggaca gggaacggagttatctgtgaaacccaatatccagaaccccgagcccgccgtgt accagctgaaggaccccagaagccaggacagcaccctgtgcctgttcaccga cttcgacagccagatcaacgtgcccaagaccatggagagcggcaccttcatca ccgacaagaccgtgctggacatgaaggccatggacagcaagagcaacggcg ccatcgcctggtccaaccagaccagcttcacatgccaggacatcttcaaggaga ccaacgccacctaccccagcagcgacgtgccctgcgacgccaccctgaccga gaagagcttcgagaccgacatgaacctgaacttccagaacctgagcgtgatgg gcctgagaatcctgctgctgaaggtggccggcttcaacctgctgatgaccctga ggctgtgggccgccagggcaaaacgttcgggttcgggtgcgccagtaaagca gacattaaactttgatttgctgaaacttgcaggtgatgtagagtcaaatccaggtc caatggcaacagggagccgaacctctctgctccttgctttcgggctcctttgccta ccgtgcctgcaggagggctcggcagggatcacccaggcaccaacatctcaga tcctggcagcaggacggcgcatgacactgagatgtacccaggatatgagacat aatgccatgtactggtatagacaagatctaggactggggctaaggctcatccatt attcaaatactgcaggtaccactggcaaaggagaagtccctgatggttatagtgt ctccagagcaaacacagatgatttccccctcacgttggcgtctgctgtaccctctc agacatctgtgtacttctgtgccagcagcctaagtttcggcactgaagctttctttg gacaaggcaccagactcacagttgtagaggacctgaggaacgtgaccccccc caaggtgtccctgttcgagcccagcaaggccgagatcgccaacaagcagaag gccaccctggtgtgcctggccaggggcttcttccccgaccacgtggagctgtct tggtgggtgaacggcaaggaggtgcacagcggcgtgagcaccgacccccag gcctacaaggagagcaactacagctactgcctgagcagcaggctgagagtga gcgccaccttctggcacaaccccaggaaccacttccgctgtcaggtgcagttcc acggcctgagcgaggaggacaagtggcccgagggcagccccaagcccgtg acccagaacatcagcgccgaggcctggggcagagccgactgcggcatcacc agcgccagctaccaccagggcgtgctgtccgccaccatcctgtacgagatcct gctgggcaaggccacactgtacgccgtgctggtgtccggcctggtgctgatgg ccatggtgaagaagaagaacagctaa | |
| 41 | atggcgacgggttcaagaacttccctacttcttgcatttggcctgctttgtttgccgt ggttacaggaagcctcagcacaacagaaggaggtggagcagaattctggacc cctcagtgttccagagggagccattgcctctctcaactgcacttacagtgaccga ggttcccagtccttcttctggtacagacaatattctgggaaaagccctgagttgat aatgttcatatactccaatggtgacaaagaagatggaaggtttacagcacagctc aataaagccagccagtatgtttctctgctcatcagagactcccagcccagtgattc agccacctacctctgtgccgtgaacttcggaggaggaaagcttatcttcggaca gggaacggagttatctgtgaaacccaatatccagaaccccgagcccgccgtgt accagctgaaggaccccagaagccaggacagcaccctgtgcctgttcaccga cttcgacagccagatcaacgtgcccaagaccatggagagcggcaccttcatca ccgacaagaccgtgctggacatgaaggccatggacagcaagagcaacggcg ccatcgcctggtccaaccagaccagcttcacatgccaggacatcttcaaggaga ccaacgccacctaccccagcagcgacgtgccctgcgacgccaccctgaccga gaagagcttcgagaccgacatgaacctgaacttccagaacctgagcgtgatgg gcctgagaatcctgctgctgaaggtggccggcttcaacctgctgatgaccctga ggctgtgggccgccagggcaaaacgttcgggttcgggtgcgccagtaaagca | The full-length nucleotide sequence of F5-KR-TCR |
| | gacattaaactttgatttgctgaaacttgcaggtgatgtagagtcaaatccaggtc caatggcaacagggagccgaacctctctgctccttgctttcgggctcctttgccta ccgtgcctgcaggagggctcggcagggatcacccaggcaccaacatctcaga tcctggcagcaggacggcgcatgacactgagatgtacccaggatatgagacat aatgccatgtactggtatagacaagatctaggactggggctaaggctcatccatt attcaaatactgcaggtaccactggcaaaggagaagtccctgatggttatagtgt ctccagagcaaacacagatgatttccccctcacgttggcgtctgctgtaccctctc agacatctgtgtacttctgtgccagcagcctaagtttcggcactgaagctttctttg gacaaggcaccagactcacagttgtagaggacctgaggaacgtgaccccccc caaggtgtccctgttcgagcccagcaaggccgagatcgccaacaagcagaag gccaccctggtgtgcctggccaggggcttcttccccgaccacgtggagctgtct tggtgggtgaacggcaaggaggtgcacagcggcgtgagcaccgacccccag gcctacaaggagagcaactacagctactgcctgagcagcaggctgagagtga gcgccaccttctggcacaaccccaggaaccacttccgctgtcaggtgcagttcc acggcctgagcgaggaggacaagtggcccgagggcagccccaagcccgtg acccagaacatcagcgccgaggcctggggcagagccgactgcggcatcacc agcgccagctaccaccagggcgtgctgtccgccaccatcctgtacgagatcct gctgggcaaggccacactgtacgccgtgctggtgtccggcctggtgctgatgg ccatggtgaagaagaagaacagctaa | |
| 42 | atggcgacgggttcaagaacttccctacttcttgcatttggcctgctttgtttgccgt ggttacaggaagcctcagcacaacagaaggaggtggagcagaattctggacc cctcagtgttccagagggagccattgcctctctcaactgcacttacagtgaccga ggttcccagtccttcttctggtacagacaatattctgggaaaagccctgagttgat aatgttcatatactccaatggtgacaaagaagatggaaggtttacagcacagctc aataaagccagccagtatgtttctctgctcatcagagactcccagcccagtgattc agccacctacctctgtgccgtgaacttcggaggaggaaagcttatcttcggaca gggaacggagttatctgtgaaacccaatatccagaaccccgagcccgccgtgt accagctgaaggaccccagaagccaggacagcaccctgtgcctgttcaccga cttcgacagccagatcaacgtgcccaagaccatggagagcggcaccttcatca ccgacaagaccgtgctggacatgaaggccatggacagcaagagcaacggcg ccatcgcctggtccaaccagaccagcttcacatgccaggacatcttcaaggaga ccaacgccacctaccccagcagcgacgtgccctgcgacgccaccctgaccga gaagagcttcgagaccgacatgaacctgaacttccagaacctgagcgtgatgg gcctgagaatcctgctgctgaaggtggccggcttcaacctgctgatgaccctga ggctgtgggccgccagggcaaaacgttcgggttcgggtgcgccagtaaagca gacattaaactttgatttgctgaaacttgcaggtgatgtagagtcaaatccaggtc caatggcaacagggagccgaacctctctgctccttgctttcgggctcctttgccta ccgtgcctgcaggagggctcggcagggatcacccaggcaccaacatctcaga tcctggcagcaggacggcgcatgacactgagatgtacccaggatatgagacat aatgccatgtactggtatagacaagatctaggactggggctaaggctcatccatt attcaaatactgcaggtaccactggcaaaggagaagtccctgatggttatagtgt ctccagagcaaacacagatgatttccccctcacgttggcgtctgctgtaccctctc agacatctgtgtacttctgtgccagcagcctaagtttcggcactgaagctttctttg gacaaggcaccagactcacagttgtagaggacctgaggaacgtgaccccccc caaggtgtccctgttcgagcccagcaaggccgagatcgccaacaagcagaag | The full-length nucleotide sequence of F5-dMUT-TCR |
| | gccaccctggtgtgcctggccaggggcttcttccccgaccacgtggagctgtct tggtgggtgaacggcaaggaggtgcacagcggcgtgagcaccgacccccag gcctacaaggagagcaactacagctactgcctgagcagcaggctgagagtga gcgccaccttctggcacaaccccaggaaccacttccgctgtcaggtgcagttcc acggcctgagcgaggaggacaagtggcccgagggcagccccaagcccgtg acccagaacatcagcgccgaggcctggggcagagccgactgcggcatcacc agcgccagctaccaccagggcgtgctgtccgccaccatcctgtacgagatcct gctgggcaaggccacactgtacgccgtgctggtgtccggcctggtgctgatgg ccatggtgaggaagaggaacagctaa | |
| 43 | atggcgacgggttcaagaacttccctacttcttgcatttggcctgctttgtttgccgt ggttacaggaagcctcagcaaaacaggaggtgacgcagattcctgcagctctg agtgtcccagaaggagaaaacttggttctcaactgcagtttcactgatagcgctat ttacaacctccagtggtttaggcaggaccctgggaaaggtctcacatctctgttg cttattcagtcaagtcagagagagcaaacaagtggaagacttaatgcctcgctg gataaatcatcaggacgtagtactttatacattgcagcttctcagcctggtgactca gccacctacctctgtgctgtgaggcccacatcaggaggaagctacatacctaca tttggaagaggaaccagccttattgttcatccgtatatccagaaccccgagcccg ccgtgtaccagctgaaggaccccagaagccaggacagcaccctgtgcctgttc accgacttcgacagccagatcaacgtgcccaagaccatggagagcggcacctt catcaccgacaagaccgtgctggacatgaaggccatggacagcaagagcaac ggcgccatcgcctggtccaaccagaccagcttcacatgccaggacatcttcaa ggagaccaacgccacctaccccagcagcgacgtgccctgcgacgccaccct gaccgagaagagcttcgagaccgacatgaacctgaacttccagaacctgagc gtgatgggcctgagaatcctgctgctgaaggtggccggcttcaacctgctgatg accctgaggctgtggagcagcagggcaaaacgttcgggttcgggtgcgccag taaagcagacattaaactttgatttgctgaaacttgcaggtgatgtagagtcaaat ccaggtccaatggcaacagggagccgaacctctctgctccttgctttcgggctc ctttgcctaccgtgcctgcaggagggctcggcaggtgtcactcagaccccaaa attccaggtcctgaagacaggacagagcatgacactgcagtgtgcccaggata tgaaccatgaatacatgtcctggtatcgacaagacccaggcatggggctgagg ctgattcattactcagttggtgctggtatcactgaccaaggagaagtccccaatg gctacaatgtctccagatcaaccacagaggatttcccgctcaggctgctgtcgg ctgctccctcccagacatctgtgtacttctgtgccagcagttacgtcgggaacac cggggagctgttttttggagaaggctctaggctgaccgtactggaggacctgag gaacgtgaccccccccaaggtgtccctgttcgagcccagcaaggccgagatc gccaacaagcagaaggccaccctggtgtgcctggccaggggcttcttccccga ccacgtggagctgtcttggtgggtgaacggcaaggaggtgcacagcggcgtg agcaccgacccccaggcctacaaggagagcaactacagctactgcctgagca gcaggctgagagtgagcgccaccttctggcacaaccccaggaaccacttccg ctgtcaggtgcagttccacggcctgagcgaggaggacaagtggcccgaggg cagccccaagcccgtgacccagaacatcagcgccgaggcctggggcagagc cgactgcggcatcaccagcgccagctaccaccagggcgtgctgtccgccacc atcctgtacgagatcctgctgggcaaggccacactgtacgccgtgctggtgtcc ggcctggtgctgatggccatggtgaagaagaagaacagctaa | The full-length nucleotide sequence of 1G4-WT-TCR |
| 44 | atggcgacgggttcaagaacttccctacttcttgcatttggcctgctttgtttgccgt | The full-length nucleotide sequence |
| | ggttacaggaagcctcagcaaaacaggaggtgacgcagattcctgcagctctg agtgtcccagaaggagaaaacttggttctcaactgcagtttcactgatagcgctat ttacaacctccagtggtttaggcaggaccctgggaaaggtctcacatctctgttg cttattcagtcaagtcagagagagcaaacaagtggaagacttaatgcctcgctg gataaatcatcaggacgtagtactttatacattgcagcttctcagcctggtgactca gccacctacctctgtgctgtgaggcccacatcaggaggaagctacatacctaca tttggaagaggaaccagccttattgttcatccgtatatccagaaccccgagcccg ccgtgtaccagctgaaggaccccagaagccaggacagcaccctgtgcctgttc accgacttcgacagccagatcaacgtgcccaagaccatggagagcggcacctt catcaccgacaagaccgtgctggacatgaaggccatggacagcaagagcaac ggcgccatcgcctggtccaaccagaccagcttcacatgccaggacatcttcaa ggagaccaacgccacctaccccagcagcgacgtgccctgcgacgccaccct gaccgagaagagcttcgagaccgacatgaacctgaacttccagaacctgagc gtgatgggcctgagaatcctgctgctgaaggtggccggcttcaacctgctgatg accctgaggctgtgggccgccagggcaaaacgttcgggttcgggtgcgccag taaagcagacattaaactttgatttgctgaaacttgcaggtgatgtagagtcaaat ccaggtccaatggcaacagggagccgaacctctctgctccttgctttcgggctc ctttgcctaccgtgcctgcaggagggctcggcaggtgtcactcagaccccaaa attccaggtcctgaagacaggacagagcatgacactgcagtgtgcccaggata tgaaccatgaatacatgtcctggtatcgacaagacccaggcatggggctgagg ctgattcattactcagttggtgctggtatcactgaccaaggagaagtccccaatg gctacaatgtctccagatcaaccacagaggatttcccgctcaggctgctgtcgg ctgctccctcccagacatctgtgtacttctgtgccagcagttacgtcgggaacac cggggagctgttttttggagaaggctctaggctgaccgtactggaggacctgag gaacgtgaccccccccaaggtgtccctgttcgagcccagcaaggccgagatc gccaacaagcagaaggccaccctggtgtgcctggccaggggcttcttccccga ccacgtggagctgtcttggtgggtgaacggcaaggaggtgcacagcggcgtg agcaccgacccccaggcctacaaggagagcaactacagctactgcctgagca gcaggctgagagtgagcgccaccttctggcacaaccccaggaaccacttccg ctgtcaggtgcagttccacggcctgagcgaggaggacaagtggcccgaggg cagccccaagcccgtgacccagaacatcagcgccgaggcctggggcagagc cgactgcggcatcaccagcgccagctaccaccagggcgtgctgtccgccacc atcctgtacgagatcctgctgggcaaggccacactgtacgccgtgctggtgtcc ggcctggtgctgatggccatggtgaagaagaagaacagctaa | of 1G4-SA-TCR |
| 45 | atggcgacgggttcaagaacttccctacttcttgcatttggcctgctttgtttgccgt ggttacaggaagcctcagcaaaacaggaggtgacgcagattcctgcagctctg agtgtcccagaaggagaaaacttggttctcaactgcagtttcactgatagcgctat ttacaacctccagtggtttaggcaggaccctgggaaaggtctcacatctctgttg cttattcagtcaagtcagagagagcaaacaagtggaagacttaatgcctcgctg gataaatcatcaggacgtagtactttatacattgcagcttctcagcctggtgactca gccacctacctctgtgctgtgaggcccacatcaggaggaagctacatacctaca tttggaagaggaaccagccttattgttcatccgtatatccagaaccccgagcccg ccgtgtaccagctgaaggaccccagaagccaggacagcaccctgtgcctgttc accgacttcgacagccagatcaacgtgcccaagaccatggagagcggcacctt catcaccgacaagaccgtgctggacatgaaggccatggacagcaagagcaac | The full-length nucleotide sequence of 1G4-KR-TCR |
| | ggcgccatcgcctggtccaaccagaccagcttcacatgccaggacatcttcaa ggagaccaacgccacctaccccagcagcgacgtgccctgcgacgccaccct gaccgagaagagcttcgagaccgacatgaacctgaacttccagaacctgagc gtgatgggcctgagaatcctgctgctgaaggtggccggcttcaacctgctgatg accctgaggctgtggagcagcagggcaaaacgttcgggttcgggtgcgccag taaagcagacattaaactttgatttgctgaaacttgcaggtgatgtagagtcaaat ccaggtccaatggcaacagggagccgaacctctctgctccttgctttcgggctc ctttgcctaccgtgcctgcaggagggctcggcaggtgtcactcagaccccaaa attccaggtcctgaagacaggacagagcatgacactgcagtgtgcccaggata tgaaccatgaatacatgtcctggtatcgacaagacccaggcatggggctgagg ctgattcattactcagttggtgctggtatcactgaccaaggagaagtccccaatg gctacaatgtctccagatcaaccacagaggatttcccgctcaggctgctgtcgg ctgctccctcccagacatctgtgtacttctgtgccagcagttacgtcgggaacac cggggagctgttttttggagaaggctctaggctgaccgtactggaggacctgag gaacgtgaccccccccaaggtgtccctgttcgagcccagcaaggccgagatc gccaacaagcagaaggccaccctggtgtgcctggccaggggcttcttccccga ccacgtggagctgtcttggtgggtgaacggcaaggaggtgcacagcggcgtg agcaccgacccccaggcctacaaggagagcaactacagctactgcctgagca gcaggctgagagtgagcgccaccttctggcacaaccccaggaaccacttccg ctgtcaggtgcagttccacggcctgagcgaggaggacaagtggcccgaggg cagccccaagcccgtgacccagaacatcagcgccgaggcctggggcagagc cgactgcggcatcaccagcgccagctaccaccagggcgtgctgtccgccacc atcctgtacgagatcctgctgggcaaggccacactgtacgccgtgctggtgtcc ggcctggtgctgatggccatggtgaggaagaggaacagctaa | |
| 46 | atggcgacgggttcaagaacttccctacttcttgcatttggcctgctttgtttgccgt ggttacaggaagcctcagcaaaacaggaggtgacgcagattcctgcagctctg agtgtcccagaaggagaaaacttggttctcaactgcagtttcactgatagcgctat ttacaacctccagtggtttaggcaggaccctgggaaaggtctcacatctctgttg cttattcagtcaagtcagagagagcaaacaagtggaagacttaatgcctcgctg gataaatcatcaggacgtagtactttatacattgcagcttctcagcctggtgactca gccacctacctctgtgctgtgaggcccacatcaggaggaagctacatacctaca tttggaagaggaaccagccttattgttcatccgtatatccagaaccccgagcccg ccgtgtaccagctgaaggaccccagaagccaggacagcaccctgtgcctgttc accgacttcgacagccagatcaacgtgcccaagaccatggagagcggcacctt catcaccgacaagaccgtgctggacatgaaggccatggacagcaagagcaac ggcgccatcgcctggtccaaccagaccagcttcacatgccaggacatcttcaa ggagaccaacgccacctaccccagcagcgacgtgccctgcgacgccaccct gaccgagaagagcttcgagaccgacatgaacctgaacttccagaacctgagc gtgatgggcctgagaatcctgctgctgaaggtggccggcttcaacctgctgatg accctgaggctgtgggccgccagggcaaaacgttcgggttcgggtgcgccag taaagcagacattaaactttgatttgctgaaacttgcaggtgatgtagagtcaaat ccaggtccaatggcaacagggagccgaacctctctgctccttgctttcgggctc ctttgcctaccgtgcctgcaggagggctcggcaggtgtcactcagaccccaaa attccaggtcctgaagacaggacagagcatgacactgcagtgtgcccaggata tgaaccatgaatacatgtcctggtatcgacaagacccaggcatggggctgagg | The full-length nucleotide sequence of 1G4-dMUT-TCR |
| | ctgattcattactcagttggtgctggtatcactgaccaaggagaagtccccaatg gctacaatgtctccagatcaaccacagaggatttcccgctcaggctgctgtcgg ctgctccctcccagacatctgtgtacttctgtgccagcagttacgtcgggaacac cggggagctgttttttggagaaggctctaggctgaccgtactggaggacctgag gaacgtgaccccccccaaggtgtccctgttcgagcccagcaaggccgagatc gccaacaagcagaaggccaccctggtgtgcctggccaggggcttcttccccga ccacgtggagctgtcttggtgggtgaacggcaaggaggtgcacagcggcgtg agcaccgacccccaggcctacaaggagagcaactacagctactgcctgagca gcaggctgagagtgagcgccaccttctggcacaaccccaggaaccacttccg ctgtcaggtgcagttccacggcctgagcgaggaggacaagtggcccgaggg cagccccaagcccgtgacccagaacatcagcgccgaggcctggggcagagc cgactgcggcatcaccagcgccagctaccaccagggcgtgctgtccgccacc atcctgtacgagatcctgctgggcaaggccacactgtacgccgtgctggtgtcc ggcctggtgctgatggccatggtgaggaagaggaacagctaa | |
| 47 | MAMVKRKDSRG | The amino acid sequence of the first wild-type (WT) human TCR β chain intracellular constant region |
| 48 | MAMVKRKDF | The amino acid sequence of the second wild-type (WT) human TCR β chain intracellular constant region |

### Example 1: Construction of TCR expression vector

Constructing F5-TCR and 1G4-TCR separately, wherein the F5-TCR includes F5-WT TCR, F5-SATCR, F5-KR TCR and F5-dMUT TCR, the 1G4-TCR includes 1G4-WT TCR, 1G4-SATCR, 1G4-KR TCR and 1G4-dMUT TCR.

As mentioned above, each F5-TCR or 1G4-TCR respectively includes a TCR α chain and a TCR β chain. Wherein the TCR α chain is formed by a human growth hormone signal peptide, a TCR α chain variable region, a TCR α chain extracellular constant region, a TCR α chain transmembrane region, and a TCR α chain intracellular region that are connected in a sequential order. The TCR β chain is formed by a human growth hormone signal peptide, a TCR β chain variable region, a TCR β chain extracellular constant region, a TCR β chain transmembrane region, and a TCR β chain intracellular region that are connected in a sequential order. The TCR α chain and the TCR β chain are linked together through F2A peptides. The amino acid or nucleotide sequences of each TCR α chain and TCR β chain mentioned above are described in the aforementioned embodiments of the present disclosure.

The amino acid sequences above, as well as the amino acid sequences with serine mutated to alanine in the TCR α chain intracellular constant region and lysine mutated to arginine in the TCR β chain constant region, were converted into base sequences after codon optimization and synthesized by the company (GenScript).

The full-length nucleotide sequences of the F5-WT TCR, F5-SA TCR, F5-KR TCR, and F5-dMUTTCR, and the 1G4-WT TCR, 1G4-SATCR, 1G4-KR TCR, and 1G4-dMUT TCR are recorded in SEQ ID NO: 39-46 respectively.

All TCR base sequences in the present disclosure are ultimately cloned into the pMSGV-LNGFR-P2A vector through enzymatic cutting and T4 ligase linkage.

### Example 2: Preparation method for retroviral packaging

The TCR expression vector (pMSGV-TCR) constructed in Example 1 is co-transfected with the envelope plasmid pHIT60/RD114 into HEK293T cells and packaging a retrovirus. Specifically, the packaging ratio system is MSGV-TCR: pHIT60: RD114=2:2:1.3, mixing well in an OPTI-DMEM medium. Adding 15.9 µl of TransIT293-Mirus transfection reagent (Mirusbio #MIR 2700), mixing well by pipetting. After standing at room temperature for 25 minutes, add the aforementioned mixed liposome liquid to a HEK293T cell culture dish and incubate at 37°C incubator for 48 hours. Then collect the virus solution and filter through 0.22 µm filter membrane and place in a -80°C refrigerator for later use.

### Example 3: Preparation and culture method of human primary mutated TCR-T Cells

Culturing human primary T cells: the human primary T cells are purchased from a commercial company (ALLCELLS). The complete culture medium for primary T cells includes 5% human serum (Gemini #100-512), RPMI-1640 medium (Hyclone #SH30809.01) coated with bispecific antibodies (Gibco #15140-122), and 1xGlutMAX (Gibco #35050-061). At the same time, 100U/ml of rhIL-2 (PeproTech #200-02) should be added to the culture medium. Resuspend human primary T cells in 1ml of the complete culture medium and place them in a 24-well plate (Nest #702001) coated with 1 µg/ml hCD3 (Biogene #317347) and 1 µg/ml hCD28 antibody (Biogene #102121). Incubate at 37°C incubator for 48 hours before conducting a virus infection experiment.

Retroviral infection of human primary T cells: Resuspend human primary T cells in a 24-well plate and extract 750µl into an EP tube (Axygen #MCT-105C). Centrifuge at 2500rpm for 5 minutes and discard the supernatant. Add 0.75µl of Polybrene (SANTA CRUZ #SC-134220), mix well by pipetting, and add to the corresponding 24-well plate. At this time, the infection system is 750µl. After centrifugation at 30°C and 2500rpm for 90 minutes, extract 500µl of supernatant from a 24-well plate and add it to an EP tube. Centrifuge at 2500rpm for 5 minutes and discard the supernatant. Add 750µl of complete T cell culture medium (1.25x rhIL-2) to a 24-well plate and incubate at 37°C incubator for 24 hours. The next day, carry out the second virus infection operation, and the specific steps are the same as the previous operation. After infection, fresh RPMI-1640 complete culture medium is supplemented every 1-2 days for primary human T cells.

### Example 4: Analysis method of flow cytometry

Flow cytometry was performed by the BD LSR Fortessa instrument (BD Bioscience) for detection. For the detection of cell surface molecules: absorb 2×10⁵ cells and place them in a 96-well U-bottom plate. Centrifuge at 1800rpm for 5 minutes and discard the supernatant. Add the flow cytometric antibody prepared with FACS buffer (1×PBS including 2% serum), and dye on ice and in the dark for 25 minutes. Wash with FACS buffer once and perform flow cytometry on the machine.

For staining of cytokines: absorb 2×10⁵ cells and place them in a 96-well U-bottom plate. Centrifuge at 1800rpm for 5 minutes and discard the supernatant. Add the flow cytometric antibody prepared with FACS buffer (1×PBS including 2% serum), and stained on ice and in the dark for 25 minutes. Wash with FACS buffer once and fix cells with polyformaldehyde fixative (Biogen #420801) for 20 minutes. Wash with FACS buffer once and add the flow cytometric antibody prepared with a membrane-breaking agent (Invitrogen #00-8333-56). Staining on ice and in the dark for 25 minutes, then wash with FACS buffer once and perform flow cytometry on the machine.

For staining of transcription factors: absorb 2×10⁵ cells and place them in a 96-well U-bottom plate. Centrifuge at 1800rpm for 5 minutes and discard the supernatant. Add the flow cytometric antibody prepared with FACS buffer (1×PBS including 2% serum), and dye on ice and in the dark for 25 minutes. Wash with FACS buffer once and fix cells with FOXP3 transcription factor fixative (Biogen #420801) for 25 minutes. Wash with FACS buffer once and add the transcription factor flow cytometric antibody prepared with a membrane-breaking agent. Dye on ice and in the dark for 25 minutes, then wash with FACS buffer once and perform flow cytometry on the machine. Data of flow cytometry is analyzed by FlowJo software (Tree Star).

### Example 5: TCR degradation induced by antigen stimulation

Experiment of downregulation of TCR expression induced by in vivo tumor antigen stimulation: On the twelfth day after the adoption of 1G4-TCR-T cells in tumor-bearing mice inoculated with K562-NYESO-1, detect the expression of TCR on the surface of TCR-T cells in spleens and tumors. As shown in Figure 1 (A), tumor antigen stimulation in tumor tissues resulted in the downregulation of TCR expression. Experiment of downregulation and degradation of TCR expression induced by *in vitro* target antigen stimulation: mix 1G4-TCR-T cells with K562-NYESO-1 target cells or K562-MART-1 non-target cells in a ratio of 1:1 in a 24-well plate and co-incubate in a 37°C incubator for 12 hours (It should be understood that the specific time is determined by different experiments). Absorb cells for flow cytometry FACS detection and detect TCR downregulation by staining TCR on the T cell surface, while TCR degradation levels are analyzed by staining intracellular TCR after cell fixation and membrane breaking. As shown in Figure 1 (B), target cell antigen stimulation promotes the downregulation and degradation of TCR. Experiment of downregulation and degradation of TCR induced by hCD3 antibody stimulation: After resuspending the activated 1G4-TCR-T cells and F5-TCR-T cells, add them to a 24-well plate coated with hCD3 antibody and co-incubate in a 37°C incubator for 12 hours (It should be understood that the specific time is determined by different experiments). Absorb cells for flow cytometry FACS detection and detect TCR downregulation by staining TCR on the T cell surface, while TCR degradation levels are analyzed by staining intracellular TCR after cell fixation and membrane breaking. As shown in Figure 1 (C, D), the CD3 antibody stimulation *in vitro* promoted the downregulation and degradation of TCR in human T cells. Comparative experiment of the expression of TCRs with different mutations: After resuspending the activated 1G4-TCR-T cells with different mutations, detecting the expression of different TCRs by FACS staining. As shown in Figure 2 (B), there is no significant difference in expression between TCRs with different mutations and wild-type TCRs. Comparative experiment of downregulation and degradation of TCR with different mutations induced by hCD3 antibody stimulation: After resuspending the activated 1G4-TCR-T cells and F5-TCR-T cells, add them to a 24-well plate coated with hCD3 antibody and co-incubate in a 37°C incubator for time required by the experiment. Detect TCR downregulation by staining TCR on the T cell surface, while TCR degradation levels are analyzed by staining intracellular TCR after cell fixation and membrane breaking. As shown in Figure 2 (C, D), compared to WT TCR, the mutant groups SA TCR, KR TCR, and dMUT TCR show more T cell membrane retention and can significantly resist downregulation and degradation caused by antibody stimulation, which was particularly evident in the dMUT TCR group. As shown in Figure (E), in the experiment of detecting surface TCR expression at different time points by the stimulation of hCD3 antibody, compared to other groups, dMUT TCR shows the strongest ability to resist degradation.

### Example 6: In vitro functional testing and construction of exhaustion model of T cells

*In vitro* functional testing of T cells: After being infected with 1G4-TCR retrovirus with different mutations, human primary T cells activated *in vitro* are cultured in RPMI-1640 complete medium for 12 days. Absorb 2×10⁵ cells into a 96-well plate and perform flow cytometry FACS detection. The detection indicators include the expression of T cell memory molecules such as CD62L TCF1, CD27, and CD45RO. As shown in Figure 3 (A-C), compared to the WT TCR group, the proportion of CD27⁺CD45RO⁺ (central memory T cells) in SA TCR, KR TCR, and dMUT TCR-T cells significantly increased, and the mutant group tended to differentiate into memory T cells. Meanwhile, the expression of memory molecules CD62L and TCF1 in T cells increased, especially in dMUT TCR-T cells.

Construction of an *in vitro* exhaustion model for T cells: After being infected with 1G4-TCR retrovirus with different mutations for 48 hours, take out human primary T cells activated *in vitro* and centrifuge at 2500rpm for 5 minutes. Resuspend TCR-T cells in a 24-well plate coated with 2µg/ml hCD3 antibodies by a complete culture medium (10⁶ cells per well), and stimulate TCR-T cells again for 48 hours. After repeating the above steps twice, end the culture and collect TCR-T cells for flow cytometry analysis. The construction of the *in vitro* exhaustion model referred to recognized relevant articles in the art (Santosha A. Vardhana et al., Nat Immunol. 2020 Sep;21(9): 1022-1033). The detection indicators are exhaustion-related molecules PD-1, TIM3, LAG-3, TOX, etc., and cytokines TNF-α and IFN-γ. As shown in Figure 3 (D-F), the expression of exhaustion-related molecules LAG-3 and TOX is significantly reduced in KR TCR-T cells and dMUT TCR-T cells, while more cytokine TNF-α and IFN-γ are produced by dMUT TCR-T cells. Overall, KR TCR-T cells and dMUT TCR-T cells can better resist the occurrence of T cell exhaustion.

### Example 7: Detection of mitochondrial function and metabolic analysis of T cells

Detection of the mitochondrial index: After being infected with 1G4-TCR retrovirus with different mutations, human primary T cells activated *in vitro* are cultured in RPMI-1640 complete medium for 12 days. Absorb 2×10⁵ cells into an EP tube, and add MitoTracker Green (Invitrogen #M7514) to dye. The final concentration is 50nM. After staining in the dark at 37°C for 1 hour, perform the flow cytometry FACS detection. As shown in Figure 4 (A), compared to WT TCR cells, there was no significant change in the number of mitochondria TCR-T cells with different mutations. The detection method for mitochondrial membrane potential is absorbing 2×10⁵ cells into an EP tube, and adding TMRE (Invitrogen #T669) to dye. The final concentration is 200nM. After staining in the dark at 37°C for 1 hour, perform the flow cytometry FACS detection. As shown in Figure 4 (B), compared to WT TCR cells, the mitochondrial membrane potential in SA TCR, KR TCR, and dMUT TCR-T cells is significantly decreased, especially in dMUT TCR-T cells. The detection method for mitochondrial reactive oxygen species is absorbing 2×10⁵ cells into an EP tube, and adding MitoSOX (Invitrogen #M36008) to dye. The final concentration is 5µM. After staining in the dark at 37°C for 1 hour, perform the flow cytometry FACS detection. As shown in Figure 4 (C), compared to WT TCR cells, the mitochondrial ROS in SA TCR, KR TCR, and mutated MUT TCR-T cells is significantly decreased. Summarizing the results of the above embodiments, different mutations of TCR do not affect the number of mitochondria in T cells, but the mitochondria in mutated TCR cells, especially dMUT TCR-T cells, exhibit a healthier state, which is related to the memory phenotype exhibited by dMUT TCR-T cells and resistance to T cell exhaustion during *in vitro* culture.

Detection of the metabolic-related indicator: human primary T cells infected with mutated 1G4-TCR retrovirus are cultured in RPMI-1640 complete medium at 37°C incubator for 12 days *in vitro.* Absorb 2×10⁵ cells into an EP tube, centrifuge at 2500rpm for 5 minutes, and discard the supernatant. Resuspend T cells in a 24-well plate by a sugar-free and serum-free medium. Use 2-NBDG (Invitrogen #N13195) with a final concentration of 50µM or 1µM of Bodipy FL C16 (Invitrogen #D3821) to treat cells. After staining in the dark at 37°C for 30 minutes, perform the flow cytometry FACS detection. Before the flow cytometry, other marker molecules can be labeled according to experiments. As shown in Figure 4 (D, E), compared to WT TCR cells, SA TCR, KR TCR, and dMUT TCR-T cells show a significant decrease in glucose intake, while there is no significant change in extracellular fatty acid intake.

### Example 8: Detection of anti-tumor function of mutated TCR-T cells

The anti-tumor experiment of mutant TCR-T cells is conducted in 4-8 weeks old immunodeficient NSG mice. To compare the better anti-tumor effect of mutant TCR-T cells with wild-type TCR-T cells, 8×10⁵ K562-NYESO-1 cells are subcutaneously inoculated into the armpits of the right hind limbs of NSG mice. After the target cells grow subcutaneously in the mice for about 8-12 days, measuring the tumor volume by a vernier caliper. Specifically, when the tumor volume is 80mm³-120mm³, NSG mice are injected with 5×10⁶ human primary T cells infected with 1G4 TCR with different mutations via tail vein injection. Measuring the tumor volume every 2 days. After approximately 14 days (depending on the experimental phenomenon), collecting the blood, spleens, and tumor tissues of NSG mice. Weigh the tumor tissues, and then use erythrocyte lysate (Biogene #420301) to lyse erythrocytes in the blood and spleen of the mice. Tumor tissues of the mice are ground and subjected by Percoll density gradient centrifugation (Cytova #17089110) to collect human primary T cells adopted from the tail vein isolated from the blood, spleens, and tumor tissues of the mice. Analyze the cells after flow cytometry staining.

The specific results are shown in Figures 5 and 6. Firstly, as shown in Figure 5 (A), through statistical analysis of tumor growth volume, compared to the WT TCR group, the experimental group of adoptive KR TCR-T cells and dMUT TCR-T cells shows a significant slowdown in tumor growth rate. As shown in Figure 5 (B), after weighing the tumor tissues, it is found that the experimental group adopting KR TCR-T cells and dMUT TCR-T cells shows a significant decrease in tumor weight. In addition, the proportion of adopted CD8⁺TCR⁻ T cells in the spleens, blood, and tumor tissues of tumor-bearing NSG mice is statistically analyzed in this embodiment. As shown in Figure 5 (C-E), compared to WT TCR-T cells, the proportion of dMUT TCR-T cells significantly increased in the spleen and blood of the mice. While in tumor tissues of the mice, compared to WT TCR-T cells, the proportion of KR TCR-T cells and dMUT TCR-T cells was significantly increased. This indicates that KR TCR-T cells and dMUT TCR-T cells have stronger anti-tumor persistence in the NSG mouse immune adoption model. As shown in Figure 6 (A), compared to wild-type TCR-T cells, further cell phenotype analysis revealed a significant increase in the proportion of CD27⁺CD45RO⁺ (central memory T cells) in SA TCR, KR TCR, and dMUT TCR-T cells in spleens. Meanwhile, as shown in Figure 6 (B, C), through the analysis of TCR-T cells in tumor tissues, it is found that compared to WT TCR-T cells, dMUT TCR-T cells express higher levels of memory molecules CD62L and CCR7. Figure 6 (D) shows that within tumor tissues, the proportion of PD-1⁺TIM-3⁺ double-positive cells in SA TCR, KR TCR, and dMUT TCR groups is significantly less than in the WT TCR group. Furthermore, as shown in Figure 6 (E-H), by detecting the fluorescence intensity of inhibitory molecules PD-1, TIM-3, LAG-3, and CD39, it is found that compared to WT TCR-T cells, the expression of inhibitory molecules in the mutated TCR-T cell group is significantly reduced. Furthermore, it is found that as shown in Figure 6 (I), the expression of key transcription factor TOX of exhaustion is significantly reduced in dMUT TCR-T cells. In summary, in the in vivo anti-tumor model, the mutated TCR-T cell groups are able to resist the occurrence of T cell exhaustion. The present disclosure also detects the production of TCR-T cytokines in tumor tissues, as shown in Figure 6 (J, K), the proportion of IFN-γ⁺CD8⁺ cells significantly increase in the adoptive KR TCR-T cell group and dMUT TCR-T cell group, indicating that KR TCR and dMUT TCR-T cells have better anti-tumor effects. Simultaneously discover the proportion of TNFα⁺IFN-γ⁺ double-positive cells in CD8⁺KR TCR-T cells and CD8⁺dMUT TCR-T cells significantly increases, indicating that KR TCR-T cells produce more cytokines than WT TCR cells, but less than dMUT TCR-T cells, indicating that dMUT TCR-T cells have better effector functions in tumor tissues.

In T cell adoptive immunotherapy, both collecting limited T cells from patients to extensively amplify TCR-T cells and the persistent decrease of T cell function and the occurrence of T cell exhaustion faced by tumor patients after adoption, are the difficulties currently faced by TCR-T cell therapy. The modification method of mutated TCR-T cells provided in the present disclosure, especially modification of dMUT TCR-T, enables TCR-T cells to exhibit more persistent anti-tumor effects and resist T cell exhaustion after amplification *in vitro* and reintroducing into a tumor model. This suggests that TCR-T cells modified by mutated dMUT TCR have the potential to achieve sustained and efficient anti-tumor functions in clinical production and treatment.

## Claims

1. An isolated T cell receptor or a fragment thereof, the T cell receptor comprises a TCR α chain and a TCR β chain, wherein the TCR β chain comprises a TCR β chain variable region and a TCR β chain constant region, the TCR β chain constant region comprises a TCR β chain extracellular constant region, a TCR β chain transmembrane region, and a TCR β chain intracellular constant region that are connected in a sequential order, and the TCR β chain intracellular constant region is selected from:
(1) a mutated TCR β chain intracellular constant region, wherein the mutated TCR β chain intracellular constant region is a wild-type TCR β chain intracellular constant region with at least one lysine mutated to arginine or alanine; or
(2) a mutated TCR β chain intracellular constant region comprising an amino acid sequence set forth in SEQ ID NO: 10 or consisting of the amino acid sequence.

2. The isolated T cell receptor according to claim 1, wherein the wild-type TCR β chain intracellular constant region is selected from:
(1) a wild-type TCR β chain intracellular constant region derived from human, mouse, or other mammalian species; or
(2) a wild-type TCR β chain intracellular constant region comprising an amino acid sequence set forth in SEQ ID NO: 9, SEQ ID NO: 47, or SEQ ID NO: 48, or consisting of the amino acid sequence.

3. The isolated T cell receptor according to any one of claims 1-2, wherein the TCR α chain comprises a TCR α chain constant region, the TCR α chain constant region comprises a TCR α chain extracellular constant region, a TCR α chain transmembrane region, and a TCR α chain intracellular constant region that are connected in a sequential order, and the TCR α chain intracellular constant region is selected from:
(1) a wild-type TCR α chain intracellular constant region derived from human, mouse, or other mammalian species;
(2) a wild-type TCR α chain intracellular constant region comprising an amino acid set forth in SEQ ID NO: 3 or consisting of the amino acid sequence;
(3) a mutated TCR α chain intracellular constant region, the mutated TCR α chain intracellular constant region is the wild-type TCR α chain intracellular constant region as described in (1) or (2) with at least one serine mutated to alanine; or
(4) a mutated TCR α chain intracellular constant region comprising an amino acid set forth in SEQ ID NO: 4 or consisting of the amino acid sequence;
optionally, the TCR α chain further comprises a TCR α chain variable region.

4. An isolated T cell receptor or a fragment thereof, the T cell receptor comprises a TCR α chain and a TCR β chain, wherein the TCR α chain comprises a TCR α chain variable region and a TCR α chain constant region, the TCR α chain constant region comprises a TCR α chain extracellular constant region, a TCR α chain transmembrane region, and a TCR α chain intracellular constant region that are connected in a sequential order, and the TCR α chain intracellular constant region is selected from:
(1) a mutated TCR α chain intracellular constant region, wherein the mutated TCR α chain intracellular constant region is a wild-type TCR α chain intracellular constant region with at least one serine mutated to alanine; or
(2) a mutated TCR α chain intracellular constant region comprising an amino acid sequence set forth in SEQ ID NO: 4 or consisting of the amino acid sequence.

5. The isolated T cell receptor according to claim 4, wherein the wild-type TCR α chain intracellular constant region is selected from:
(1) a wild-type TCR α chain intracellular constant region derived from human, mouse, or other mammalian species; or
(2) a wild-type TCR α chain intracellular constant region comprising an amino acid sequence set forth in SEQ ID NO: 3 or consisting of the amino acid sequence.

6. The isolated T cell receptor according to any one of claims 4-5, wherein the TCR β chain comprises a TCR β chain constant region, the TCR β chain constant region comprises a TCR β chain extracellular constant region, a TCR β chain transmembrane region, and a TCR β chain intracellular constant region that are connected in a sequential order, and the TCR β chain intracellular constant region is selected from:
(1) a wild-type TCR β chain intracellular constant region derived from human, mouse, or other mammalian species;
(2) a wild-type TCR β chain intracellular constant region comprising an amino acid sequence set forth in SEQ ID NO: 9, SEQ ID NO: 47, or SEQ ID NO: 48, or consisting of the amino acid sequence;
(3) a mutated TCR β chain intracellular constant region, wherein the mutated TCR β chain intracellular constant region is the wild-type TCR α chain intracellular constant region as described in (1) or (2) with at least one lysine mutated to arginine or alanine; or
(4) a mutated TCR β chain intracellular constant region comprising an amino acid sequence set forth in SEQ ID NO: 10 or consisting of the amino acid sequence;
optionally, the TCR β chain further comprises a TCR β chain variable region.

7. The isolated T cell receptor according to any one of the claims, wherein the isolated T cell receptor recognizes polypeptide antigens, lipid antigens, and polysaccharide antigens.

8. The isolated T cell receptor according to any one of the claims, wherein the isolated T cell receptor recognizes tumor antigens, microbial antigens, or autoantigens.

9. The isolated T cell receptor according to claim 8, wherein the isolated T cell receptor recognizes one or more antigens of BCMA, CA9, CTAG, CCL-1, CSPG4, EGFR, EPG-2, EPG-40, FCRL5, FBP, OGD2, GPC3, GPRC5D, HER3, HER4, HLA-A1, HLA-A2, LRRC8A, CMV, MUC1, MUC16, MART-1, NCAM, PRAME, PSCA, PSMA, ROR1, TPBG, TAG72, TRP1, TRP2, VEGFR, VEGFR2, WT-1, MAGE-A1/A3/A4/A6/A10/C2, gp100, CEA, NYESO-1, AFP, MART-1, HERV-E, HER2, LMP1/2, BRLF-1, BMLF-1, HPV-16E6/E7, KRAS G12D, KRAS G12V, TP53 R175H, β-GlcCer, eLPA, LPE, CA199, CA72-4, or CA125; preferably is one or more antigens of MAGE-A1/A3/A4/A6/A10/C2, gp100, CEA, NYESO-1, AFP, MART-1, HERV-E, HER2, LMP1/2, BRLF-1, BMLF-1, HPV-16E6/E7, KRAS G12D, KRAS G12V, TP53 R175H, β-GlcCer, eLPA, LPE, CA199, CA72-4, or CA125.

10. The isolated T cell receptor according to any one of the claims, wherein the TCR β chain variable region is selected from:
(1) the TCR β chain variable region comprising three complementarity determining regions (CDR) comprised in an amino acid sequence set forth in SEQ ID NO: 17;
(2) the TCR β chain variable region comprising three complementarity determining regions (CDR) comprised in an amino acid sequence set forth in SEQ ID NO: 25;
(3) the TCR β chain variable region comprising CDR1 of an amino acid sequence set forth in SEQ ID NO: 18, CDR2 of an amino acid sequence set forth in SEQ ID NO: 19, and/or CDR3 of an amino acid sequence set forth in SEQ ID NO: 20;
(4) the TCR β chain variable region comprising CDR1 of an amino acid sequence set forth in SEQ ID NO: 26, CDR2 of an amino acid sequence set forth in SEQ ID NO: 27, and/or CDR3 of an amino acid sequence set forth in SEQ ID NO: 28;
(5) the TCR β chain variable region comprising an amino acid sequence set forth in SEQ ID NO: 17 or consisting of the amino acid sequence; or
(6) the TCR β chain variable region comprising an amino acid sequence set forth in SEQ ID NO: 25 or consisting of the amino acid sequence.

11. The isolated T cell receptor according to any one of the claims, wherein the TCR α chain variable region is selected from:
(1) the TCR α chain variable region comprising three complementarity determining regions (CDR) comprised in an amino acid sequence set forth in SEQ ID NO: 13;
(2) the TCR α chain variable region comprising three complementarity determining regions (CDR) comprised in an amino acid sequence set forth in SEQ ID NO: 21;
(3) the TCR α chain variable region comprising CDR1 of an amino acid sequence set forth in SEQ ID NO: 14, CDR2 of an amino acid sequence set forth in SEQ ID NO: 15, and/or CDR3 of an amino acid sequence set forth in SEQ ID NO: 16;
(4) the TCR α chain variable region comprising CDR1 of an amino acid sequence set forth in SEQ ID NO: 22, CDR2 of an amino acid sequence set forth in SEQ ID NO: 23, and/or CDR3 of an amino acid sequence set forth in SEQ ID NO: 24;
(5) the TCR α chain variable region comprising an amino acid sequence set forth in SEQ ID NO: 13 or consisting of the amino acid sequence; or
(6) the TCR α chain variable region comprising an amino acid sequence set forth in SEQ ID NO: 21 or consisting of the amino acid sequence.

12. The isolated T cell receptor according to any one of the claims, further comprising one or more of the following features:
(1) the TCR α chain extracellular constant region is a TCR α chain extracellular constant region derived from human, mouse, or other mammalian species;
(2) the TCR α chain extracellular constant region comprises an amino acid sequence set forth in SEQ ID NO: 1, or comprises an amino acid sequence with 1, 2, 3, 4, 5, 6, 7, 8, 9, or 10 mutations compared to the amino acid sequence set forth in SEQ ID NO: 1, or comprises an amino acid sequence with at least 90%, 91%, 92%, 93%, 94%, 95%, 96%, 97%, 98%, or 99% homology with the amino acid sequence set forth in SEQ ID NO: 1, or consists of the amino acid sequence;
(3) the TCR α chain transmembrane region is a TCR α chain transmembrane region derived from human, mouse, or other mammalian species;
(4) the TCR α chain transmembrane region comprises an amino acid sequence set forth in SEQ ID NO: 2, or comprises an amino acid sequence with 1, 2, 3, 4, 5, 6, 7, 8, 9, or 10 mutations compared to the amino acid sequence set forth in SEQ ID NO: 2, or comprises an amino acid sequence with at least 90%, 91%, 92%, 93%, 94%, 95%, 96%, 97%, 98%, or 99% homology with the amino acid sequence set forth in SEQ ID NO: 2, or consists of the amino acid sequence;
(5) the TCR β chain extracellular constant region is a TCR β chain extracellular constant region derived from human, mouse, or other mammalian species;
(6) the TCR β chain transmembrane region comprises an amino acid sequence set forth in SEQ ID NO: 7, or comprises an amino acid sequence with 1, 2, 3, 4, 5, 6, 7, 8, 9, or 10 mutations compared to the amino acid sequence set forth in SEQ ID NO: 7, or comprises an amino acid sequence with at least 90%, 91%, 92%, 93%, 94%, 95%, 96%, 97%, 98%, or 99% homology with the amino acid sequence set forth in SEQ ID NO: 7, or consists of the amino acid sequence;
(7) the TCR β chain transmembrane region is a TCR β chain transmembrane region derived from human, mouse, or other mammalian species; or
(8) the TCR β chain transmembrane region comprises an amino acid sequence set forth in SEQ ID NO: 8, or comprises an amino acid sequence with 1, 2, 3, 4, 5, 6, 7, 8, 9, or 10 mutations compared to the amino acid sequence set forth in SEQ ID NO: 8, or comprises an amino acid sequence with at least 90%, 91%, 92%, 93%, 94%, 95%, 96%, 97%, 98%, or 99% homology with the amino acid sequence set forth in SEQ ID NO: 8, or consists of the amino acid sequence.

13. The isolated T cell receptor according to any one of the claims, wherein the TCR α chain constant region and TCR β chain constant region are selected from:
(1) the TCR α chain constant region comprising an amino acid sequence set forth in SEQ ID NO: 5 or consisting of the amino acid sequence, and the TCR β chain constant region comprising an amino acid sequence set forth in SEQ ID NO: 12 or consisting of the amino acid sequence;
(2) the TCR α chain constant region comprising an amino acid sequence set forth in SEQ ID NO: 6 or consisting of the amino acid sequence, and the TCR β chain constant region comprising an amino acid sequence set forth in SEQ ID NO: 12 or consisting of the amino acid sequence; or
(3) the TCR α chain constant region comprising an amino acid sequence set forth in SEQ ID NO: 6 or consisting of the amino acid sequence, and the TCR β chain constant region comprising an amino acid sequence set forth in SEQ ID NO: 11 or consisting of the amino acid sequence.

14. The isolated T cell receptor according to any one of the claims, further comprising a signal peptide, wherein the signal peptide forms a signal peptide-variable region structure with the TCR α chain variable region and/or the TCR β chain variable region.

15. The isolated T cell receptor according to claim 14, wherein the signal peptide is a human growth hormone signal peptide, a CD8α signal peptide, or an immunoglobulin signal peptide.

16. An isolated nucleic acid or fragment thereof encoding the isolated T cell receptor or fragment thereof according to any one of the claims.

17. A nucleic acid construct, comprising the isolated nucleic acid or fragment thereof according to claim 16.

18. A vector, comprising the nucleic acid construct according to claim 17.

19. The vector according to claim 18, wherein the vector is an expression vector or a crisper gene editing vector, preferably a retroviral vector.

20. An engineered cell, comprising the nucleic acid construct according to claim 17 or the vector according to any one of claims 18-19.

21. The engineered cell according to claim 20, wherein the engineer cell is a T cell, such as a human T cell.

22. A method for producing the engineered cell, comprising
(1) transforming or transducing host cells by the nucleic acid construct according to claim 17 or the vector according to any one of claims 18-19;
(2) culturing the host cells in an appropriate culture medium;
(3) separating and purifying the engineered cells from the culture medium or the host cells.

23. An application of the isolated T cell receptor or fragment thereof, the nucleic acid or fragment thereof, the nucleic acid construct, the vector, or the engineered cell according to any one of the claims in any one or more of the following uses:
(1) an application of inhibiting the T cell receptor degradation during a process of antigen stimulation;
(2) providing a killing effect on tumors or inhibiting tumor growth;
(3) maintaining the proliferation ability of T cells;
(4) preparation of anti-tumor immune drugs and cells.

24. A method for modifying a T cell receptor, comprising:
(1) mutating at least one lysine in a wild-type TCR β chain intracellular constant region to arginine or alanine; and/or
(2) mutating at least one serine in a wild-type TCR α chain intracellular constant region to alanine.

25. The method for modifying the T cell receptor according to claim 24, wherein:
(1) the wild-type TCR β chain intracellular constant region is selected from the wild-type TCR β chain intracellular constant region according to claim 2, and/or
(2) the wild-type TCR α chain intracellular constant region is selected from the wild-type TCR α chain intracellular constant region according to claim 5.
